# EUROPEAN PATENT APPLICATION

(11) **EP 3 029 627 A1**
(43) Date of publication of application: **08.06.2016**
(21) Application number: 14832094.8
(22) Date of filing: 30.07.2014
(51) Int. Cl.: G06Q 50/22

(54) **MEDICAL SUPPORT SERVER AND MEDICAL SUPPORT SYSTEM**

(30) Priority: 31.07.2013 JP 2013159931
(71) Applicant: FUJIFILM CORPORATION, Minato-ku Tokyo 106-8620 (JP)
(72) Inventor: UEDA, Satoshi, Tokyo 106-8620 (JP); OHTA, Yasunori, Ashigarakami-gun Kanagawa 258-8538 (JP); USAMI, Ryosuke, Tokyo 106-8620 (JP)
(74) Representative: Klunker . Schmitt-Nilson . Hirsch
(86) International application number: PCT/JP2014/070033
(87) International publication number: WO 2015/016250

(57) **Abstract**

In an initial step of emergency in which a patient (P) is transported from a site (15) to a hospital (12A), a medical support system (19) creates general-purpose emergency timeline information (FTLD) that is available in the initial step of emergency of a plurality of diseases according to treatment start of a paramedic (F) for the patient (P), and manages medical care information of the patient (P) based on the created emergency timeline information (FTLD). After specifying of disease of the patient (P), the medical support system (19) creates dedicated timeline information (ETLD) corresponding to the specified disease, and manages the medical care information of the patient (P) based on the created dedicated timeline information (ETLD). The medical care information managed using the emergency timeline information (FTLD) transitions to the dedicated timeline information (ETLD).

## Description

### CROSS-REFERENCE TO RELATED APPLICATIONS

This application is a Continuation of PCT International Application No. PCT/JP2014/070033 filed on July 30, 2014, which claims priority under 35 U.S.C §119(a) to Japanese Patent Application No. 2013-159931 filed July 31, 2013. The above application is hereby expressly incorporated by reference, in its entirety, into the present application.

### BACKGROUND OF THE INVENTION

### 1. Field of the Invention

The present invention relates to a medical support server and a medical support system for supporting emergency medical care.

### 2. Description Related to the Prior Art

Conventionally, an emergency medical system in which, when an emergency arises in which a patient requires medical care from a doctor due to sudden illness, injury, or the like, a paramedic is dispatched to a site where the patient is located, and the patient is transported from the site which is a transport source to a medical facility which is a transport destination in which medical care of the patient is performed, is provided. In the medical facility, an emergency medical team including a plurality of medical staff, such as doctors or nurses as members, is on stand-by. The medical team medically examines and treats a transported patient in cooperation with each member. Thus, in modern emergency medical care, since the medical care of the patient is performed by a medical team, it is preferable that medical care information on medical examination or treatment given to the patient can be shared among the members of the medical team.

A medical support system in which medical care information of a patient can be shared between a plurality of medical staff is described in JP2012-027565A (US Patent Application Publication No. 2012/0022885). This medical support system includes a plurality of client terminals respectively used by a plurality of medical staff, and a medical support server that creates timeline information of a patient and distributes the timeline information to each client terminal. The timeline information is information for managing various types of medical care information regarding medical examination, inspection, treatment, or the like of the patient along a time axis. The timeline information is created in the medical support server when a patient with a cerebrovascular disorder such as a stroke is transported to a hospital and capturing of an inspection image or the like is performed. According to the medical support system, the medical care information of the patient can be shared between a plurality of medical staff by distribution of the timeline information. Further, a temporal flow regarding medical care of the patient can be easily recognized from the timeline information.

A medical support system in which treatment plan information in which a scheduled medical care item required for medical care is set along a time axis for each type of disease is standardized for each disease by a medical support server, and the treatment plan information is distributed from this medical support server to a plurality of client terminals used by a doctor, a nurse, or the like is described in JP2003-108661A. Performance of the performed scheduled medical care item is recorded in the treatment plan information. According to this medical support system, since the performance of the scheduled medical care item corresponding to the medical care information is registered in the treatment plan information, it is possible to share the medical care information of a patient among a plurality of doctors or nurses. Further, it is possible to easily recognize a temporal flow regarding medical care of the patient based on the scheduled medical care item set along the time axis, and the performance thereof.

The timeline information described in JP2012-027565A and the treatment plan information described in JP2003-108661A are also useful in emergency medical care for various diseases since medical care information of a patient can be shared among a plurality of doctors or nurses and a temporal flow regarding the medical care of the patient can also be easily recognized. There is a description in JP2012-027565A that a technology can be applied to diseases other than cerebrovascular disorder, but a method of creating the timeline information available to the emergency medical care for other diseases is not specifically described. Further, in the medical support system disclosed in JP2003-108661A, since the treatment plan information is standardized for each disease, the medical support system can cope with various types of diseases. However, treatment plan information corresponding to a step before disease is specified, that is, an initial step of emergency in which a patient is transported from a site to the hospital is not included.

### SUMMARY OF THE INVENTION

An object of the present invention is to provide a medical support server and a medical support system in which timeline information is available in an initial step of emergency in which a patient is transported from a site to a hospital.

In order to solve the above problems, a medical support server of the present invention supports medical care for a patient based on timeline information for managing medical care information of the patient and a scheduled medical care item required for medical care of the patient along a time axis. The medical support server includes a timeline creation unit and a medical care information transition unit. The timeline creation unit creates, as the timeline information, emergency timeline information used in an initial step of emergency in which the patient is transported from a site as a transport source to a medical facility as a transport destination. Further, the timeline creation unit creates dedicated timeline information of specified disease of the patient in the case where the disease of the patient is specified. The medical care information transition unit makes a transition of management of the medical care information of the patient from the emergency timeline information to the dedicated timeline information in the case where the dedicated timeline information is created after the creation of the emergency timeline information of the patient.

It is preferable that in the emergency timeline information, a medical care item performed in common in the initial step of emergency of a plurality of types of diseases is set as the scheduled medical care item.

It is preferable that a medical care item that is treated by a paramedic is set in the scheduled medical care item of the emergency timeline information.

It is preferable that in the dedicated timeline information, an item of medical care to be performed for each type of disease is set as the scheduled medical care item.

It is preferable that in the scheduled medical care item of the dedicated timeline information, at least one of a type and content of a medical care item is different according to the type of the disease.

It is preferable that a plurality of types of dedicated timeline information in which thrombolytic therapy is set in common, as the scheduled medical care item, includes a treatment available time of the thrombolytic therapy and a dose of a thrombolytic drug different according to a type of disease.

It is preferable that the plurality of types of dedicated timeline information include at least first dedicated timeline information corresponding to cerebrovascular disease, and second dedicated timeline information corresponding to cardiovascular disease.

It is preferable that in the case where the emergency timeline information is used for childbirth, the timeline creation unit creates, as the dedicated timeline information, maternal timeline information used for management of the medical care information of a mother, and neonatal timeline information used for management of the medical care information of a newborn.

It is preferable that the timeline creation unit creates the emergency timeline information and the dedicated timeline information based on an emergency format in which a medical care item to be performed in common in the initial step of emergency of a plurality of types of diseases is set as the scheduled medical care item, and a plurality of types of dedicated formats in which a medical care item to be performed for each type of disease is set as the scheduled medical care item.

It is preferable that the medical support server further includes a medical care information registration unit for receiving the medical care information of the patient and registering the received medical care information in the emergency timeline information or the dedicated timeline information.

It is preferable that the medical care information registration unit receives the medical care information transmitted from a medical device used by a paramedic to treat the patient in the initial step of emergency, and registers the received medical care information in the emergency timeline information.

It is preferable that the medical care information registration unit receives the medical care information from an in-hospital information server for managing medical care information of the patient in the medical facility to which the patient is transported after the initial step of emergency ends, and registers the received medical care information in the dedicated timeline information.

It is preferable that the medical support server further includes a distribution unit for distributing the emergency timeline information and the dedicated timeline information to a client terminal over a communication network.

It is preferable that the medical support server further includes a timeline transfer unit for transferring all or part of a management function of managing the timeline information to another server after the initial step of emergency ends.

It is preferable that the timeline transfer unit transfers a function of creating the dedicated timeline information among functions of the timeline creation unit, and a function of the medical care information transition unit to the other server.

A medical support system of the present invention includes a medical support server that supports medical care for a patient based on timeline information for managing medical care information of the patient and a scheduled medical care item required for medical care of the patient along a time axis. As described above, the medical support server includes the timeline creation unit, the medical care information transition unit, and the timeline transfer unit. A transport destination server is included in the medical support system, and the timeline transfer unit transfers all or part of a management function of the timeline information to the transport destination server.

It is preferable that the transport destination server is an in-hospital information server that is managed in a medical facility as the transport destination of the patient.

According to the present invention, since it is possible to create the emergency timeline information in which the scheduled medical care item to be performed in the initial step of emergency is set, it is possible to register the medical care information of the patient in the initial step of emergency in the emergency timeline information. Accordingly, since it is possible to view the medical care information of the patient and the scheduled medical care item in the initial step of emergency based on the emergency timeline information, it is possible to appropriately diagnose the patient in the initial step of emergency. Further, after specifying of disease of the patient, the dedicated timeline information of the disease only is created and the management of medical care information transitions from the emergency timeline information to the dedicated timeline information. Accordingly, it is possible to view the medical care information of the patient and the scheduled medical care item from the initial step of emergency to the specifying of the disease based on the dedicated timeline information, and perform medical care.

### BRIEF DESCRIPTION OF DRAWINGS

For more complete understanding of the present invention, and the advantage thereof, reference is now made to the subsequent descriptions taken in conjunction with the accompanying drawings, in which:
Fig. 1 is a schematic diagram of an emergency medical system;
Fig. 2 is a conceptual diagram illustrating a creation timing of timeline information;
Fig. 3 is a schematic diagram illustrating a configuration of a medical support system;
Fig. 4 is a block diagram illustrating a configuration of a computer used for a medical support server;
Fig. 5 is a block diagram illustrating a functional configuration of a medical support system;
Fig. 6 is a block diagram illustrating a functional configuration of a timeline management unit;
Fig. 7 is a conceptual diagram illustrating transition from emergency timeline information to dedicated timeline information;
Fig. 8 is an illustrative diagram illustrating a configuration of triage information;
Fig. 9 is an illustrative view illustrating a configuration of a format DB;
Fig. 10 is an illustrative view illustrating a configuration of a timeline DB;
Fig. 11 is an illustrative diagram illustrating a configuration of an emergency timeline screen;
Fig. 12 is an illustrative diagram illustrating a configuration of the emergency timeline screen on which triage information is displayed;
Fig. 13 is an illustrative diagram illustrating a configuration of an emergency timeline screen in which an ultrasonic image is displayed;
Fig. 14 is an illustrative diagram illustrating a configuration of an emergency timeline screen in which a disease input window is displayed;
Fig. 15 is an illustrative diagram illustrating a configuration of an emergency timeline screen in which a comment input window is displayed;
Fig. 16 is an illustrative diagram illustrating a configuration of a timeline screen in which an operation termination registration window is displayed;
Fig. 17 is an illustrative diagram illustrating an operation termination screen of the timeline information;
Fig. 18 is an illustrative diagram illustrating a configuration of a dedicated timeline screen;
Fig. 19 is a flowchart illustrating a procedure of distributing a temporary patient ID;
Fig. 20 is a flowchart illustrating a procedure of creating the emergency timeline information;
Fig. 21 is a flowchart illustrating a procedure of creating the dedicated timeline information;
Fig. 22 is a flowchart illustrating an operation termination procedure for the timeline information;
Fig. 23 is a block diagram illustrating a configuration of an emergency portable terminal having a function of inputting a patient ID;
Fig. 24 is a conceptual diagram illustrating transition from emergency timeline information to dedicated timeline information in a second embodiment;
Fig. 25 is a block diagram illustrating a configuration of a medical support server of a third embodiment;
Fig. 26 is a flowchart illustrating a procedure of transferring a function of managing timeline information; and
Fig. 27 is a conceptual diagram regarding transfer of the function of managing timeline information.

### DESCRIPTION OF THE PREFERRED EMBODIMENTS

### [First Embodiment]

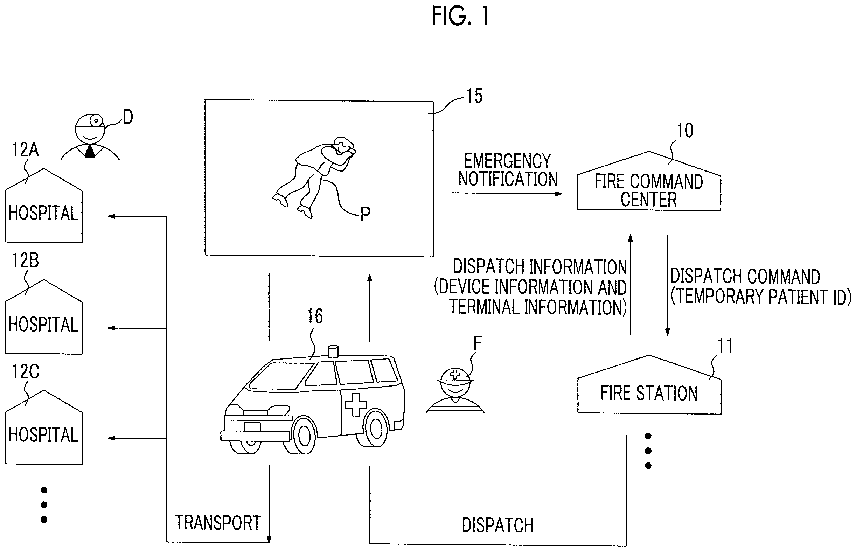

An emergency medical system in which, when an emergency patient requiring medical care of a doctor is generated due to sudden disease, injury, or the like, a paramedic is dispatched to a site where the patient is located, and the patient is transported from the site to a medical facility in which medical care of the patient is performed, is provided in each municipality of an administrative district or the like. As illustrated in Fig. 1, this emergency medical system is built by, for example, a fire command center 10 that is a facility playing a central role in the emergency medical care in the area, a plurality of fire stations 11, and a plurality of hospitals that are medical facilities, such as a hospital 12A, a hospital 12B, and a hospital 12C.

The fire command center 10 receives an emergency notification from the patient P who is in a site 15, specifies an address of the site 15 from notification content, and instructs the fire station 11 closest to the site 15 to dispatch paramedics. The fire station 11 organizes the paramedics including a plurality of paramedics F, including emergency response personnel, and dispatches an ambulance 16 in response to the dispatch command. After arriving at the site 15, the paramedics perform emergency treatment on the patient P, place the patient P in the ambulance 16, and transport the patient P in any one of the hospitals 12A to 12C. The emergency response personnel is a paramedic who has acquired an emergency response personnel qualification, and can perform ensuring of a vein path, tracheal intubation, drug administration, and treatment using a semi-automatic defibrillator on a patient in a cardiopulmonary arrest state under instructions of the doctor.

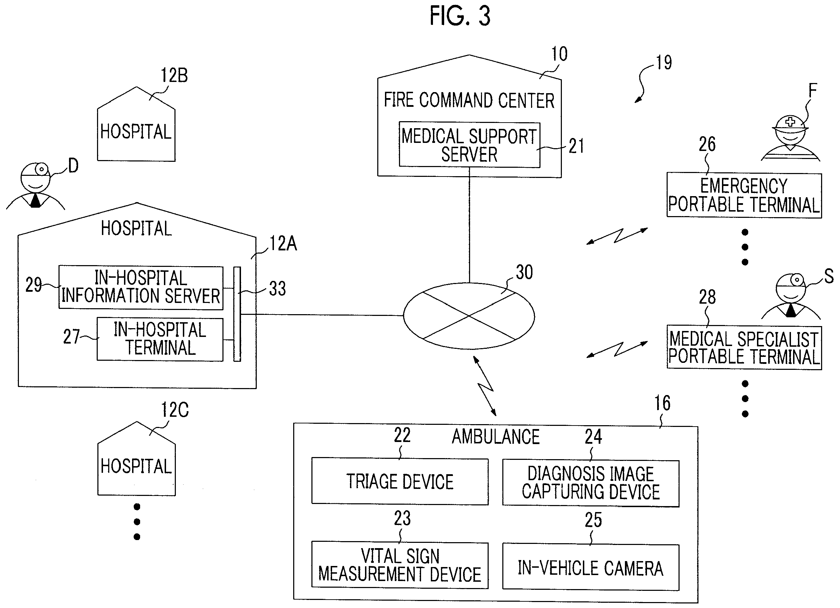

The hospitals 12A to 12C are emergency designated hospitals including an emergency medical facility, and an emergency medical team, including a plurality of medical staffs such as doctors D or nurses as members, is waiting. The medical team medically examines and treats the transported patient P in conjunction with each member. Further, in the emergency medical system, medical specialists S (see Fig. 3) with expert knowledge of disease in the specific field may be added in order to deal with the disease in the specific field that is difficult to be dealt with by the medical team.

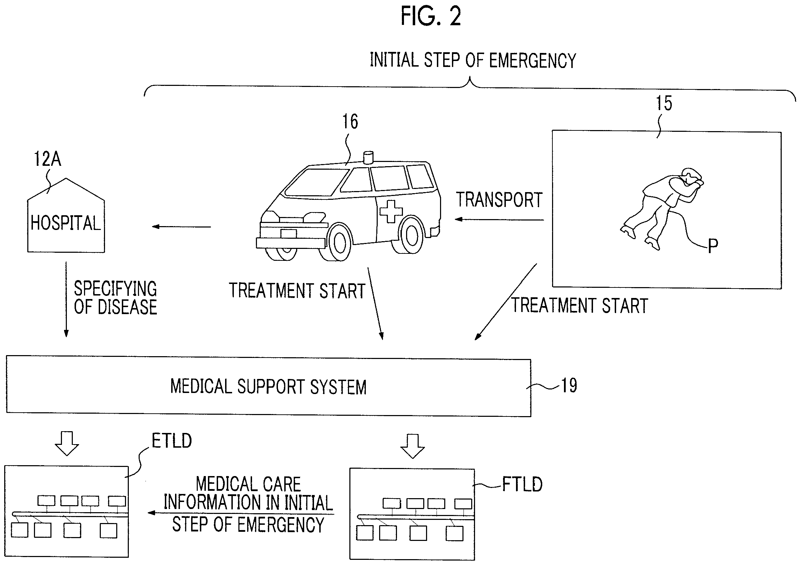

As illustrated in Fig. 2, a medical support system 19 of the present invention is a computer system that supports medical care for emergency patients using the timeline information for managing the medical care information of the patient P and the scheduled medical care item of the patient P along and the time axis. The medical support system 19 creates emergency timeline information FTLD used for general purpose in the initial step of emergency of a plurality of types of diseases according to start of treatment for the patient P in the initial step of emergency in which the patient P is transported from the site 15 to any one of the hospitals 12A to 12C. Further, when the disease of the patient P is specified, dedicated timeline information ETLD of the specified disease only is created, and management of the medical care information transitions from the emergency timeline information FTLD to the dedicated timeline information ETLD. The medical care information is information on medical examination, inspection, treatment, or the like subjected to the patient P, and includes inspection images. Further, the scheduled medical care item is an item of diagnosis or treatment required when medical care is performed on the patient P.

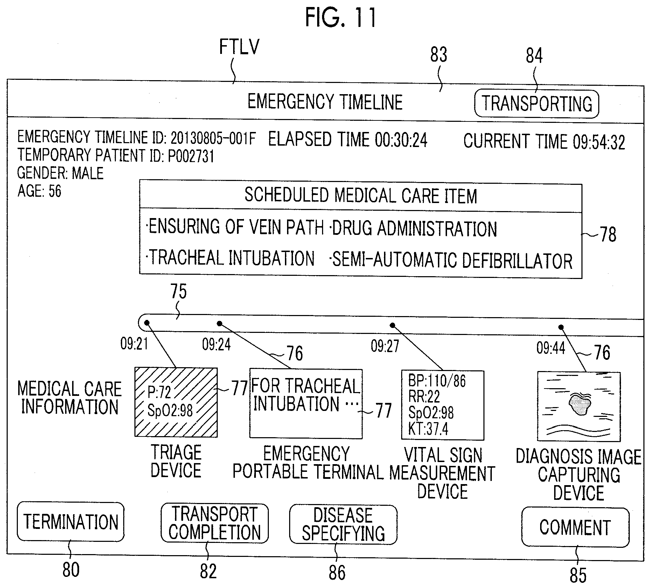

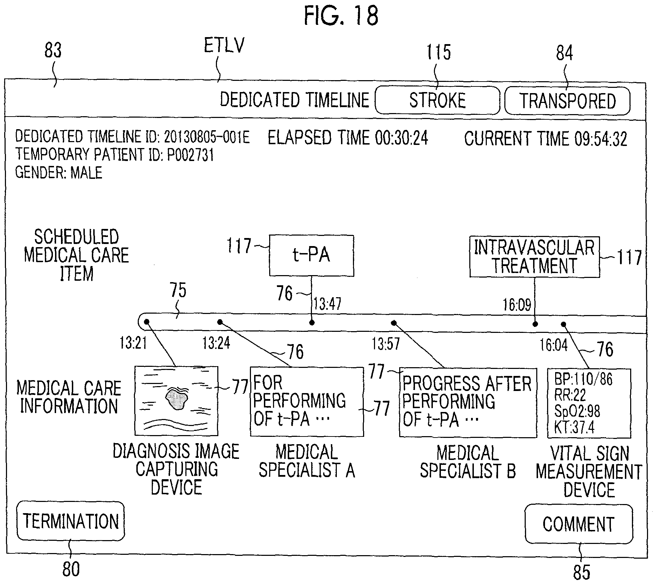

The emergency timeline information FTLD and the dedicated timeline information ETLD are distributed to the client terminal that is used by the doctor D or the like. The distributed emergency timeline information FTLD and the distributed dedicated timeline information ETLD are displayed as an emergency timeline screen FTLV (see Fig. 11) and a dedicated timeline screen ETLV (see Fig. 18) on the display of the client terminal.

In the emergency timeline screen FTLV and the dedicated timeline screen ETLV, a strip time bar 75 indicating the lapse of time from medical care start for the patient P is displayed in a horizontal direction. In the time bar 75, the time regresses to the left of the timeline screen, and arrives at a creation time of the timeline information. Further, in the time bar 75, the time progresses to the right of the timeline screen, and arrives at a current time.

A plurality of information display frames 77 are arranged along the time bar 75 over and under the time bar 75 of the emergency timeline screen FTLV. Various medical care information on medical care subjected to the patient P is displayed in an information display frame 77. Further, a plurality of schedule display frames 117 are arranged along the time bar 75 over and under the time bar 75 of the dedicated timeline screen ETLV. Preset scheduled medical care item is displayed in the schedule display frame 117.

The information display frame 77 and the schedule display frame 117 are connected to the time bar 75 by a lead line 76. Each lead line 76 is connected to a position on the time bar 75 corresponding to a generation time of the medical care information displayed in the information display frame 77, and a position on the time bar 75 corresponding to a scheduled time of the scheduled medical care item displayed in the schedule display frame 117. Therefore, it is possible to easily understand a temporal course of the medical care and the scheduled time from the timeline information.

As illustrated in Fig. 3, the medical support system 19 includes, for example, a medical support server 21, a triage device 22, a vital sign measurement device 23, a diagnosis image capturing device 24, an in-vehicle camera 25, an emergency portable terminal 26, an in-hospital terminal 27, a medical specialist portable terminal 28, and an in-hospital information server 29. Each device constituting the medical support system 19 is connected by a communication network 30. The communication network 30 includes the Internet or a mobile communication network.

The medical support server 21 is installed in, for example, the fire command center 10. The medical support server 21 creates the emergency timeline information FTLD of the patient P when receiving the treatment start information indicating that treatment of the patient P has started in an initial step of emergency. Further, when the medical support server 21 receives the disease specifying information indicating that the disease of the patient P has been specified, from the in-hospital terminal 27 of hospital to which the patient P has been transported, the medical support server 21 creates the dedicated timeline information ETLD and makes a transition of the management of the medical care information in the initial step of emergency registered in the emergency timeline information FTLD to the dedicated timeline information ETLD. The medical support server 21 distributes the created emergency timeline information FTLD and the created dedicated timeline information ETLD to the emergency portable terminal 26, the in-hospital terminal 27, and the medical specialist portable terminal 28, which are client terminals.

The medical support server 21 receives medical care information of the patient P generated after the creation of the emergency timeline information FTLD, and registers the received medical care information in the emergency timeline information FTLD. Further, after the creation of the dedicated timeline information ETLD, the medical support server 21 requests the in-hospital information server 29 in any of the hospitals 12A to 12C to which the patient P has been transported, to distribute the medical care information and the diagnosis image of the patient P, and registers the medical care information and the diagnosis image distributed from the in-hospital information server 29 in the dedicated timeline information ETLD in response to the distribution request. The emergency timeline information FTLD and the dedicated timeline information ETLD in which the medical care information has been registered are redistributed to the client terminals 26 to 28.

The triage device 22, the vital sign measurement device 23, the diagnosis image capturing device 24 , and the in-vehicle camera 25 are medical devices used by the paramedic F in order to treat the patient P, and are mounted on the ambulance 16. The triage device 22, the vital sign measurement device 23, the diagnosis image capturing device 24, and the in-vehicle camera 25 are used for treatment of the patient P by the paramedic F to generate the medical care information of the patient P and transmit the generated medical care information to the medical support server 21. The medical support server 21 identifies the medical care information that is first received among the plurality of medical care information transmitted from the triage device 22, the vital sign measurement device 23, the diagnosis image capturing device 24, and the in-vehicle camera 25, as the treatment start information described above, and starts creation of the timeline information.

The triage device 22 is a device that displays a degree of severity indicating urgency of the medical care for the patient P. The triage device 22 is mounted within the ambulance 16, or on the body of the patient P at the site 15 brought out from the ambulance 16. Conventionally, triage work of classifying patients based on a degree of severity and displaying the degree of severity using a triage tag attached to the body of the patient in order to determine a priority of care when a large number of patients are generated in large-scale disaster is known. The triage device 22 is used in place of a conventional triage tag, and includes a setting function of setting the degree of severity, a function of displaying the set degree of severity, and a transmission function of transmitting triage information including the set degree of severity as medical care information of the patient P to the medical support server 21.

The vital sign measurement device 23 is a bio-monitor which is installed in the ambulance 16, and measures vital signs such as blood pressure, a breathing rate, a blood oxygen saturation level, body temperature, and an electrocardiogram of the patient P carried into the ambulance 16. The vital sign measurement device 23 transmits vital information including measurement results of the vital signs as the medical care information of the patient P to the medical support server 21.

The diagnosis image capturing device 24 is, for example, an ultrasonic imaging device which is installed in the ambulance 16, and captures an ultrasonic image of the patient P carried into the ambulance 16. The diagnosis image capturing device 24 transmits ultrasonic image data of the patient P to the medical support server 21 as medical care information of the patient P. In addition to the ultrasonic imaging device, an X-ray imaging device, a computed tomography (CT) imaging device, or the like may be used as the diagnosis image capturing device 24.

The in-vehicle camera 25 is installed on, for example, a ceiling of the ambulance 16, and captures a still image or a moving image of the patient P carried into the ambulance 16 so that the image is provided for diagnosis of the patient P. Therefore, the in-vehicle camera 25 is included in the diagnosis image capturing device which is one medical device of the present invention. The in-vehicle camera 25 transmits in-vehicle imaging data including data of a still image or a moving image of the patient P as medical care information of the patient P to the medical support server 21.

The emergency portable terminal 26 is a portable terminal carried by each of a plurality of paramedics F organized as paramedics at the time of dispatching. A so-called smart phone is used. The emergency portable terminal 26 is used for, for example, inquiry about advice regarding emergency treatment, or inquiry about whether a patient is allowed to be transported to the hospitals 12A to 12C based on a phone function of the smart phone. Further, the emergency portable terminal 26 is also used for, for example, capturing of a still image or a moving image of the site 15 or the patient P, and recording of interview with the patient P or a witness through a photographing function and a recording function of the smart phone. Portable imaging data including a still image, a moving image, or audio data is transmitted to the medical support server 21 as the medical care information of the patient P.

The emergency portable terminal 26 is also used for viewing of the emergency timeline information FTLD and the dedicated timeline information ETLD distributed from the medical support server 21, and inputting of a comment for the emergency timeline information FTLD and the dedicated timeline information ETLD. For the input of the comment, a text input function of the smart phone is used. The comment input to the emergency portable terminal 26 is transmitted to the medical support server 21 as the medical care information of the patient P. Further, when transport of the patient P to the hospital is complete, the emergency portable terminal 26 transmits transport completion information indicating that the transport of the patient P to the hospital has been completed to the medical support server 21.

In the hospitals 12A to 12C, respective hospital information systems that manage various types of medical care information in the hospitals are provided. The hospital information system includes the in-hospital information server 29, at least one in-hospital terminal 27, and a hospital network 33 that connects the in-hospital information server 29 to the in-hospital terminal 27. The hospital network 33 is built as an Intranet using the same communication protocol as that of the Internet, and is connected to the communication network 30 via a firewall (not illustrated).

The in-hospital information server 29 is a computer system that manages electronic medical chart information and a diagnosis image of the patient P. The electronic medical chart information is stored in an electronic medical chart database (not illustrated; a database is hereinafter referred to as a DB) for each patient. The electronic medical chart information includes, for example, personal information and medical care information of the patient P. The personal information includes, for example, a patient name, an in-hospital patient ID, date of birth, a gender, and an address. The medical care information is information on medical examination, inspection, and treatment performed on the patient P in the hospital, and includes, for example, a medical care date, a medical department, an injury name, a diagnosis result, a type or an amount of medication, and a prescription pharmacy name.

The in-hospital information server 29 receives the electronic medical chart information created by the in-hospital terminal 27, and stores the electronic medical chart information in the electronic medical chart database. Further, the in-hospital information server 29 searches for the electronic medical chart DB in response to a reading request from the in-hospital terminal 27 or the medical support server 21, and transmits the searched electronic medical chart information to the in-hospital terminal 27 or the medical support server 21.

A diagnosis image capturing device such as an X-ray imaging device, a CT imaging device, a magnetic resonance imaging (MRI) imaging device, an ultrasonic imaging device, and an endoscope is connected to the in-hospital information server 29. The in-hospital information server 29 receives a diagnosis image captured by the diagnosis image capturing device, and stores the diagnosis image in a diagnosis image DB. The diagnosis image stored in the diagnosis image DB is associated with an in-hospital patient ID such that a patient corresponding to the diagnosis image can be identified. Further, the in-hospital information server 29 searches for the diagnosis image DB in response to a distribution request from the in-hospital terminal 27 or the medical support server 21, and transmits the searched diagnosis image to the in-hospital terminal 27 or the medical support server 21.

The in-hospital terminal 27 is a client terminal that is installed in each medical department in a hospital 12A, and is operated by a doctor D or a nurse in each medical department. The in-hospital terminal 27 is used, for example, for input of the electronic medical chart information to the in-hospital information server 29, viewing of the electronic medical chart information and the diagnosis image, and imaging reservation of the diagnosis image for the diagnosis image capturing device.

Further, the in-hospital terminal 27 is also used for viewing of the emergency timeline information FTLD and the dedicated timeline information ETLD distributed from the medical support server 21, and input of a comment to the emergency timeline information FTLD and the dedicated timeline information ETLD. A comment input to the in-hospital terminal 27 is transmitted to the medical support server 21 as the medical care information of the patient P. Further, if the disease of the patient P has been identified, the hospital terminal 27 of the hospital to which the patient P has been transported transmits disease specifying information indicating that the disease of the patient P has been specified to the medical support server 21. A disease name of the patient P is included in the disease specifying information.

Since the in-hospital information server and the in-hospital terminal provided in the hospitals 12B and 12C have the same configuration as that of the in-hospital information server 29 and the in-hospital terminal 27 in the hospital 12A, detailed description thereof will be omitted.

The medical specialist portable terminal 28 is a portable terminal that is carried by a medical specialist S which is registered in the medical support server 21 in advance. A so-called smart phone is used. The specialist S is a doctor with expert knowledge of the specific field such as cerebrovascular disease or cardiovascular disease and provides, for example, advice regarding treatment or an opinion regarding medical care for a critically ill patient, to the paramedic F, the doctor D of the hospital, or the like. The medical specialist portable terminal 28 is also used for viewing the emergency timeline information FTLD and the dedicated timeline information ETLD distributed from the medical support server 21, and inputting of a comment to the emergency timeline information FTLD and the dedicated timeline information ETLD. Advice regarding treatment or an opinion regarding the medical care is provided by the comment to the timeline information by the medical specialist S. A comment input to the medical specialist portable terminal 28 is transmitted to the medical support server 21 as the medical care information of the patient P.

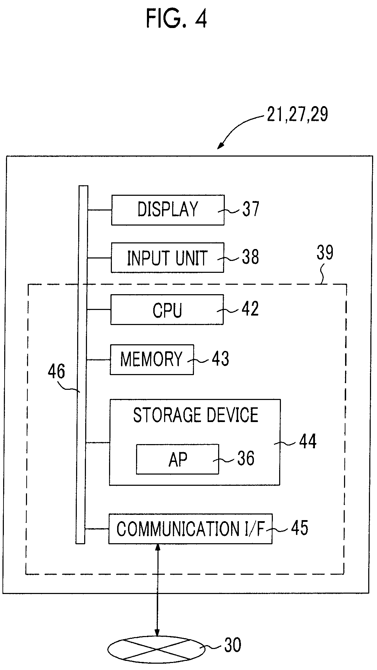

As illustrated in Fig. 4, the medical support server 21, the in-hospital terminal 27, and the in-hospital information server 29 are constituted by a computer such as a personal computer or a workstation. The computer operates a control program such an operating system or an application program (AP) 36 for causing the computer to function as a desired server or terminal, so as to function as the medical support server 21, the in-hospital terminal 27, and the in-hospital information server 29. The computer constituting the medical support server 21, the in-hospital terminal 27, and the in-hospital information server 29 includes a display 37, an input unit 38 such as a keyboard or a mouse, and a computer main body 39.

A central processing unit (CPU) 42, a memory 43, a storage device 44, and a communication I/F 45 are provided in the computer main body 39, and are connected to each other via a data bus 46. The storage device 44 is a device that stores various data and includes, for example, a hard disk drive. An AP 36 such as a control program, a medical support program, and various databases is stored in the storage device 44.

The memory 43 is a work memory used for the CPU 42 to execute a process. The CPU 42 generally controls each unit of the computer by loading the control program stored in the storage device 44 into the memory 43 and executing the process according to the program. The communication I/F 45 is a communication interface for connecting to the communication network 30 and communicating with each device of the medical support system 19.

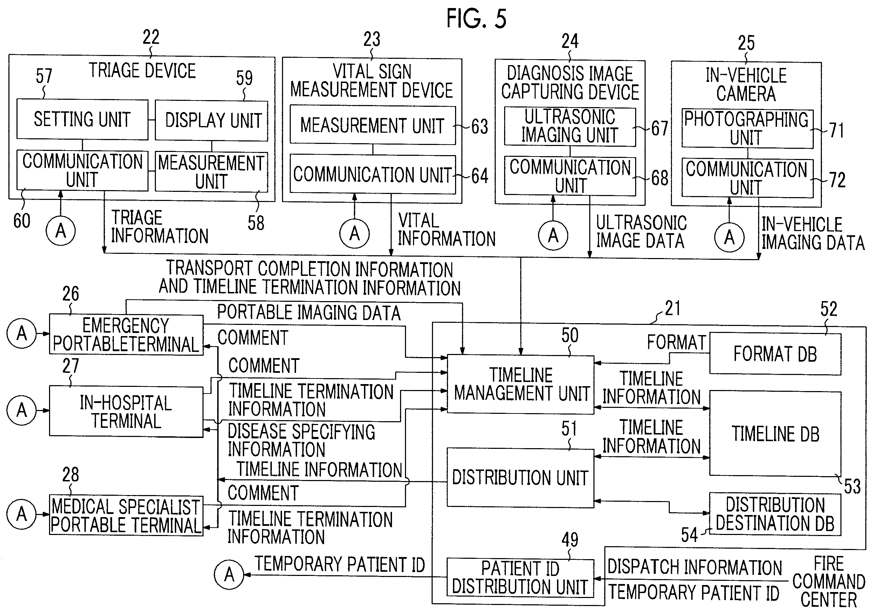

As illustrated in Fig. 5, when the medical support program starts up, the CPU 42 of the medical support server 21 functions as a patient ID distribution unit 49, a timeline management unit 50, and a distribution unit 51 in cooperation with the memory 43. Further, a format DB 52 that stores format information of the emergency timeline information FTLD and the dedicated timeline information ETLD, a timeline DB 53 that stores the emergency timeline information FTLD and the dedicated timeline information ETLD, and a distribution destination DB 54 in which distribution information on client terminals 26 to 28 are stored in the storage device 44 of the medical support server 21.

The patient ID distribution unit 49 distributes the temporary patient ID of the patient P to the respective medical devices 22 to 25 and the respective client terminals 26 to 28. The temporary patient ID distributed from the patient ID distribution unit 49 is transmitted together with the medical care information when the medical care information of the patient is transmitted from each of the medical devices 22 to 25 and each of the client terminals 26 to 28 to the medical support server 21.

In the medical support system 19, for example, when a large number of patients are generated by an accident, a disaster, or the like, a plurality of emergency timeline information FTLD is created for each patient, and accordingly, it is necessary for the patient corresponding to medical care information transmitted from each of the medical devices 22 to 25 and each of the client terminals 26 to 28 to be identified. Therefore, the patient ID distribution unit 49 distributes the temporary patient ID for identifying the medical care information to the medical device and the client terminal, and the medical device and the client terminal transmits the medical care information together with the temporary patient ID when transmitting the medical care information to the medical support server 21.

The distribution of the temporary patient ID is performed, for example, in the following procedure. A fire emergency system (not illustrated) that performs a dispatch command or the like in the fire command center 10 imparts a temporary patient ID to the patient P according to emergency notification, and transmits a dispatch command including the temporary patient ID to the fire station 11. A dispatch processing system (not illustrated) that processes the dispatch command in the fire station 11 determines the ambulance 16 to be dispatched, organizes paramedics, and then, transmits dispatch information including device information of each of the medical devices 22 to 25 mounted on the ambulance 16, terminal information of the emergency portable terminal 26 of each paramedic F to be dispatched, and the like to the fire emergency system. The fire emergency system receiving the dispatch information from the dispatch processing system transmits the temporary patient ID and the dispatch information corresponding to the temporary patient ID to the medical support server 21 over the communication network 30.

The device IDs of the medical devices 22 to 25 and communication information necessary for communication with the medical devices 22 to 25 are included in the device information in the dispatch information described above. Further, the terminal ID of the emergency portable terminal 26 and communication information necessary for communication with the emergency portable terminal 26 are included in the terminal information of the dispatch information. For example, an IP address or a MAC address imparted to each of the medical devices 22 to 25 and the emergency portable terminal 26 is included in the communication information of the medical devices 22 to 25 and the emergency portable terminal 26. When the patient ID and the dispatched information are transmitted from the fire emergency system to the medical support server 21, emergency notification reception time, gender and age or year of the patient P may be transmitted together.

The patient ID distribution unit 49 distributes the temporary patient ID to each of the medical devices 22 to 25 and each emergency portable terminal 26 based on the communication information included in the dispatch information. Further, the patient ID distribution unit 49 also distributes the temporary patient ID to each in-hospital terminal 27 and each medical specialist portable terminal 28 registered in the distribution destination DB 54. Each of the medical devices 22 to 25 and each of the client terminals 26 to 28 receiving the temporary patient ID impart the temporary patient ID to the medical care information when transmitting the medical care information to the medical support server 21. Accordingly, the medical support server 21 can identify a patient corresponding to the received medical care information based on the temporary patient ID.

The in-hospital information server 29 of the hospital to which the patient P has been transported imparts the in-hospital patient ID used in the hospital to the patient P, and transmits the in-hospital patient ID to the medical support server 21. The medical support server 21 registers the in-hospital patient ID in the timeline information of the patient P, and manages the timeline information of the patient P based on the in-hospital patient ID after transport to the hospital.

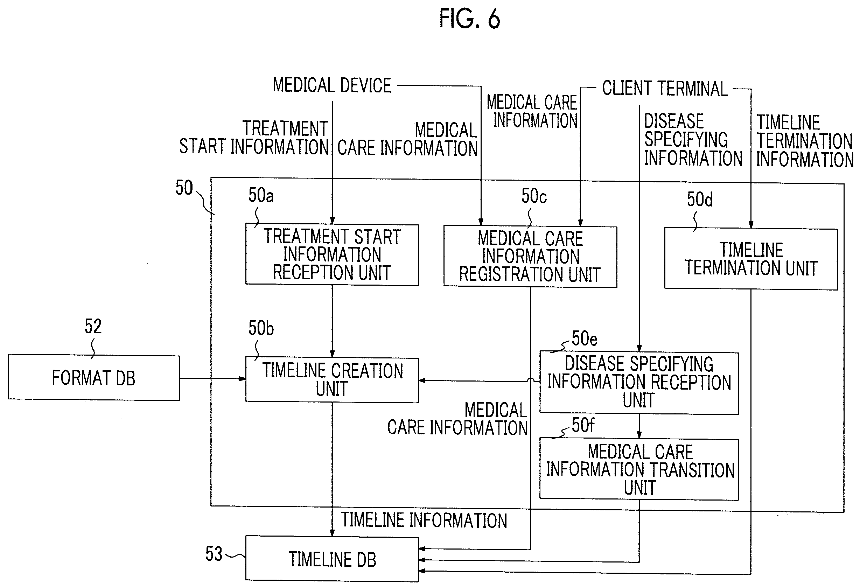

As illustrated in Fig. 6, the timeline management unit 50 generally manages the timeline information, such as the creation of the emergency timeline information FTLD and the dedicated timeline information ETLD, the registration of the medical care information in the emergency timeline information FTLD and the dedicated timeline information ETLD, and transition of management of the medical care information from the emergency timeline information FTLD to the dedicated timeline information ETLD. The timeline management unit 50 functions as a treatment start information reception unit 50a, a timeline creation unit 50b, a medical care information registration unit 50c, a timeline termination unit 50d, a disease specifying information reception unit 50e, and a medical care information transition unit 50f.

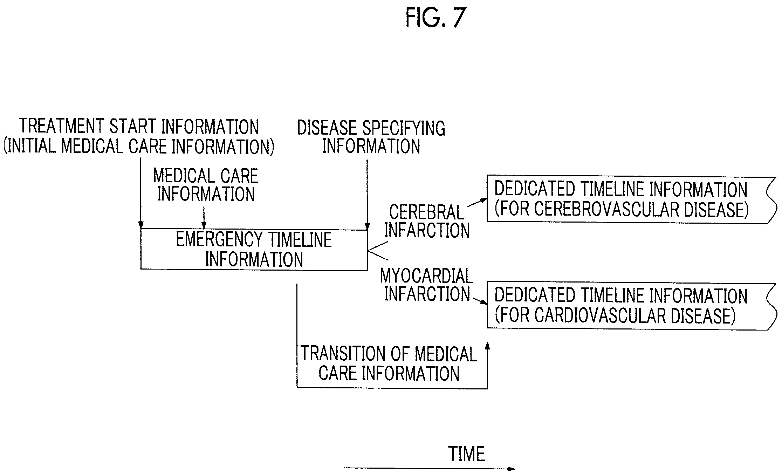

The treatment start information reception unit 50a receives, as the treatment start information, first medical care information that is acquired from the patient P by the medical device such as the triage device 22, the vital sign measurement device 23, the diagnosis image capturing device 24, and the in-vehicle camera 25, as illustrated in Fig. 7. Therefore, even when the timeline management unit 50 receives a plurality of medical care information from the respective medical devices 22 to 25, the treatment start information reception unit 50a does not receive, as treatment start information, medical care information received at a second or subsequent time.

When receiving the medical care information to be handled as the treatment start information, the timeline creation unit 50b creates one piece of emergency timeline information FTLD for each temporary patient ID imparted to the received medical care information. Therefore, when a plurality of treatment start information with different temporary patient IDs is received, a plurality of emergency timeline information FTLD corresponding to each temporary patient ID is created. Accordingly, the same number of emergency timeline information FTLD as the number of patients is created, and accordingly, it is possible to prevent mismanagement of medical care information such as medical care information of one patient being managed in a plurality of emergency timeline information FTLD, and medical care information of a plurality of patients being managed in one piece of emergency timeline information FTLD.

The timeline creation unit 50b reads the emergency format that is a template of the emergency timeline information FTLD corresponding to a degree of severity included in the triage information from the format DB 52, registers the medical care information received as the treatment start information and the temporary patient ID in the read emergency format, and creates the emergency timeline information FTLD. The timeline management unit 50 imparts an emergency timeline ID to the created emergency timeline information FTLD and stores the resultant emergency timeline information FTLD in the timeline DB 53.

When the medical care information is received from each of the medical devices 22 to 25 and each of the client terminals 26 to 28 after the emergency timeline information FTLD is created, the medical care information registration unit 50c confirms the temporary patient ID in the received medical care information, and registers the received medical care information in the emergency timeline information FTLD corresponding to the temporary patient ID. Further, when the patient P is transported to the hospital, the medical care information registration unit 50c designates the in-hospital patient ID of the patient P, requests the in-hospital information server 29 of the hospital to which the patient P has been transported to distribute the medical care information and the diagnosis image, and registers, in the timeline information, the medical care information and the diagnosis image distributed from the in-hospital information server 29 in response to the distribution request.

The disease specifying information reception unit 50e receives the disease specifying information indicating that the disease of the patient P has been identified, from the in-hospital terminal 27 of the hospitals 12A to 12C to which the patient P has been transported. When the disease specifying information is received, the timeline creation unit 50b creates one piece of the dedicated timeline information ETLD for each temporary patient ID imparted to the received disease specifying information.

The timeline creation unit 50b reads a dedicated format that is a template of the dedicated timeline information ETLD corresponding to the disease name included in the disease specifying information from the format DB 52. The timeline creation unit 50b registers a temporary patient ID in the read dedicated format to create dedicated timeline information ETLD. For example, the timeline creation unit 50b creates dedicated timeline information ETLD corresponding to cerebrovascular disease when the disease name is stroke, and creates dedicated timeline information ETLD corresponding to cardiovascular disease when the disease name is myocardial infarction. The timeline creation unit 50b imparts a dedicated timeline ID to the created dedicated timeline information ETLD and stores the resultant dedicated timeline information ETLD in the timeline DB 53.

The medical care information transition unit 50f makes a transition of the management of the medical care information registered in the emergency timeline information FTLD to the dedicated timeline information ETLD having the same temporary patient ID. The dedicated timeline information ETLD manages the medical care information transitioning from the emergency timeline information FTLD along a time axis.

The timeline termination unit 50d terminates the operation of the timeline information when the timeline information is not required due to a condition of the patient being mild, or when the timeline information is no longer required due to patient's recovery, death, or the like. The timeline termination unit 50d performs a termination process when a terminating operation is performed in the medical support server 21, or when timeline termination information is transmitted from the client terminal, such as the emergency portable terminal 26, the in-hospital terminal 27, and the medical specialist portable terminal 28. In this termination process, "operation termination" and a reason for operation termination are registered as operation status indicating operational situation of the timeline information in the timeline information of the patient that is a termination target. For the timeline information in which the operation status is "operation termination", the registration of the medical care information is then completed and an operation termination screen is distributed to the client terminals 26 to 28.

The distribution unit 51 reads the emergency timeline information FTLD and the dedicated timeline information ETLD stored in the timeline DB 53, creates an emergency timeline screen FTLV and a dedicated timeline screen ETLV based on the read emergency timeline information FTLD and the read dedicated timeline information ETLD, and distributes the emergency timeline screen FTLV and the dedicated timeline screen ETLV to the client terminals 26 to 28 based on the distribution information stored in the distribution destination DB 54. Communication information required to distribute the timeline information to each emergency portable terminal 26, each in-hospital terminal 27, and each medical specialist portable terminal 28 is registered in the distribution information. Since treatment of an initial step of emergency for the patient P terminates after transport of the patient P to the hospital, the distribution unit 51 terminates the distribution of the medical care information to the emergency portable terminal 26 after the transport of the patient P to the hospital. Further, when the operation of the timeline information terminates in the timeline termination unit 50d, the distribution unit 51 distributes the operation termination screen to the client terminals 26 to 27.

The triage device 22 includes a setting unit 57 that sets a degree of severity of the patient P, a measurement unit 58 that measures a vital sign of the patient P, a display unit 59 that displays the degree of severity and a result of the measurement, and a communication unit 60 that creates triage information including the degree of severity and the measurement result and transmits the triage information to the medical support server 21 as the medical care information of the patient P.

The setting unit 57 and the display unit 59 are constituted by, for example, a touch panel using a color liquid crystal panel. The paramedic F generally determines the degree of severity of the patient P using a determination method called the START method, and operates the touch panel to set the degree of severity. In the START method, the degree of severity of the patient is classified into four steps: "deceased, or life-saving is impossible", "serious life-threatening state", "not in a serious life-threatening state, but transport is necessary", and "emergency transport is not necessary" based on whether or not a patient can walk, a breathing state, a breathing rate, a circulation state, and a level of consciousness. Four identification colors including black, red, yellow, and green are assigned to each degree of severity. An identification mark of the identification color corresponding to the degree of severity set by the setting unit 57 is displayed on the touch panel.

The measurement unit 58 has the same function as a pulse oximeter that is conventionally used in the medical field. The measurement unit 58 irradiates the body such as an ear or a finger tip of the patient P with red light and infrared light from a light emitting unit built into a mounting unit mounted on a body such as an ear or a fingertip of the patient P, and receives light transmitted through or reflected by the body of the patient P using a light reception unit built into the mounting unit. The measurement unit 58 measures a pulse and a blood oxygen saturation level based on an amount of the light received by the light reception unit.

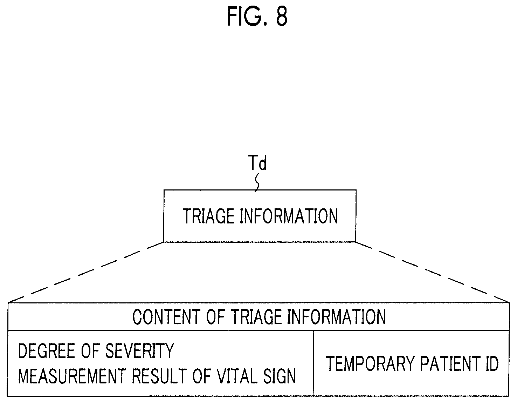

The communication unit 60 functions as the patient ID reception unit of the present invention, and receives the temporary patient ID from the patient ID distribution unit 49 of the medical support server 21. Further, the communication unit 60 functions as a treatment start information transmission unit which transmits the treatment start information indicating that the treatment for the patient P has started to the medical support server 21. As illustrated in Fig. 8, the communication unit 60 creates triage information Td as the medical care information also handled as the treatment start information. The degree of severity set by the setting unit 57, and the measurement result of the vital sign measured by the measurement unit 58 are included in the triage information Td. Further, the temporary patient ID received from the patient ID distribution unit 49 is added to the triage information Td.

The vital sign measurement device 23 includes a measurement unit 63 that measures vital signs such as blood pressure, breathing rate, blood oxygen saturation level, body temperature, and electrocardiogram of the patient P, and a communication unit 64 that transmits the vital information including measurement results of the vital signs to the medical support server 21 as the medical care information of the patient P. The communication unit 64 also functions as a patient ID reception unit and a treatment start information transmission unit, similar to the communication unit 60 of the triage device 22. Further, the temporary patient ID is added to the vital information, similar to the triage information Td.

The diagnosis image capturing device 24 includes an ultrasonic imaging unit 67 that captures an ultrasonic image of the patient P, and a communication unit 68 that transmits ultrasonic image data of the patient P as the medical care information of the patient P to the medical support server 21. The communication unit 68 also functions as a patient ID reception unit and a treatment start information transmission unit, similar to the communication unit 60 of the triage device 22. Further, the temporary patient ID is added to the ultrasonic image data, similar to the triage information Td.

The in-vehicle camera 25 includes a photographing unit 71 that captures a still image or a moving image of the patient P carried into the ambulance 16, and a communication unit 72 that transmits in-vehicle imaging data including data of the still image or the moving image of the patient P as the medical care information of the patient P to the medical support server 21. The communication unit 72 also functions as a patient ID reception unit and a treatment start information transmission unit, similar to the communication unit 60 of the triage device 22. Further, the temporary patient ID is added to the in-vehicle imaging data, similar to the triage information Td.

The temporary patient ID is also imparted to the portable imaging data and the comment transmitted as the medical care information from the emergency portable terminal 26, the in-hospital terminal 27, and the medical specialist portable terminal 28 to the medical support server 21. Therefore, the medical support server 21 can specify the patient corresponding to the comment by confirming the temporary patient ID.

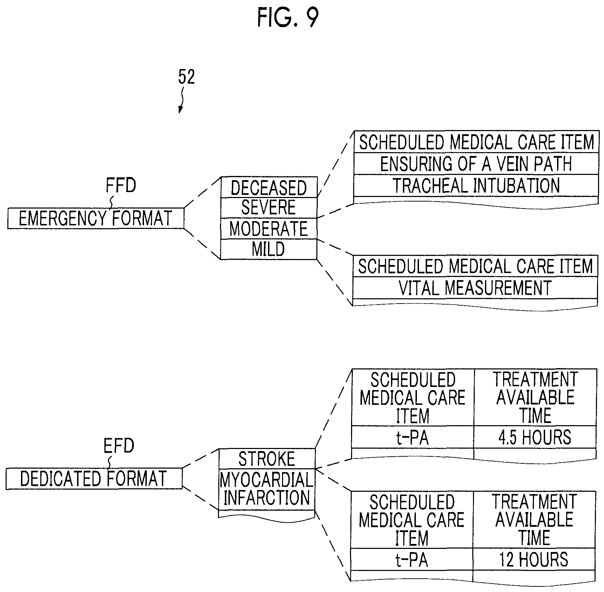

As illustrated in Fig. 9, an emergency format FFD and a dedicated format EFD are stored in the format DB 52. For the emergency format FFD, for example, four types including "death", "severe", "moderate" and "mild" are provided to correspond to the four steps of the degree of severity set in the triage device 22. The timeline management unit 50 reads the emergency format FFD corresponding to the degree of severity included in the triage information Td from the format DB 52 when creating the emergency timeline information FTLD.

For example, "vital measurement" is registered as the scheduled medical care item in the "mild" of the emergency format FFD. Further, ensuring of a vein path, tracheal intubation, drug administration, and treatment using a semi-automatic defibrillator for which treatment by the emergency response personnel can be performed are registered in the "severe" of the emergency format FFD.

For the dedicated format EFD, a plurality of types such as "stroke" and "myocardial infarction" are provided for each disease. When the timeline management unit 50 creates the dedicated timeline information ETLD, the timeline management unit 50 reads the dedicated format EFD corresponding to a disease name included in the disease specifying information from the format DB 52.

For example, "thrombolytic therapy (t-PA)" is registered as the scheduled medical care item in the "stroke" of the dedicated format EFD. "4.5 hours" from disease generation is registered as "treatment available time" in which administration of thrombolysis is available, in the scheduled medical care item "thrombolytic therapy (t-PA)". Further, "thrombolytic therapy (t-PA)" is registered as the scheduled medical care item in the "myocardial infarction" of the dedicated format EFD. "12 hours" from the disease generation is registered as the "treatment available time" in the scheduled medical care item "thrombolytic therapy (t-PA)" of the myocardial infarction. Thus, even with the same scheduled medical care item, the treatment available time set according to a type of disease is different.

Since treatment to be performed on the patient P in the initial step of emergency is treatment intended to be performed at a treatment available timing, which is determined by the paramedic F, such as vital measurement or capturing of the ultrasonic image, the "treatment available time" is not set in the scheduled medical care item of the emergency format FFD.

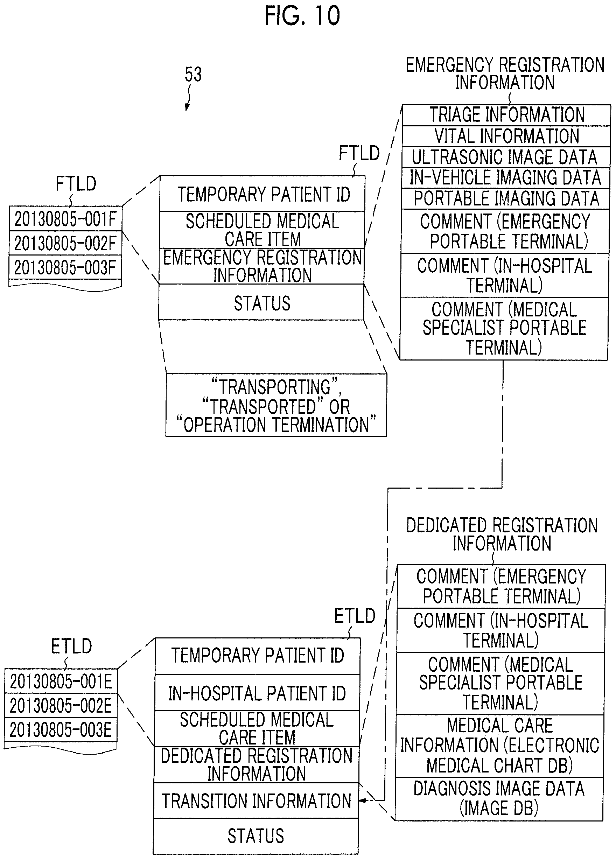

As illustrated in Fig. 10, a plurality of emergency timeline information FTLD to which the emergency timeline ID is imparted, and a plurality of dedicated timeline information ETLD to which the dedicated timeline ID is imparted are stored in the timeline DB 53. The emergency timeline ID includes, for example, a date on which the timeline information has been created, identification information for identifying a plurality of timeline information created on the creation date, and a code "F" indicating emergency. For example, "20130805-001F" indicates that the emergency timeline information is emergency timeline information initially created on August 5, 2013.

The emergency timeline information FTLD includes the temporary patient ID, a scheduled medical care item, emergency registration information, and the status. A medical care item set in the emergency format FFD is set in the scheduled medical care item. Medical care information registered by the timeline management unit 50 in the initial step of emergency is stored in the emergency registration information. Specifically, triage information, vital information, ultrasonic image data, and in-vehicle imaging data received from the triage device 22, the vital sign measurement device 23, the diagnosis image capturing device 24, and the in-vehicle camera 25, respectively, are registered in the emergency registration information. Further, the portable photographing data and the comment received from each emergency portable terminal 26, and the comment received from each of the in-hospital terminal 27 and the medical specialist portable terminal 28 are registered in the emergency registration information.

"Transporting" indicating that the patient P is being transported to the hospital, "transported" indicating that the transport to the hospital has been completed, and "operation termination" indicating that the operation of the timeline information has terminated are registered in the status. The timeline creation unit 50b registers "transporting" in the status at the time of creating the timeline information. The medical care information registration unit 50c registers "transported" in the status when receiving the transport completion information from the emergency portable terminal 26. Further, the timeline termination unit 50d registers "operation termination" in the status when a terminating operation is performed in the medical support server 21 or when timeline termination information is received from the client terminal, such as the emergency portable terminal 26, the in-hospital terminal 27, and the medical specialist portable terminal 28.

The dedicated timeline ID includes the same number string as that of the emergency timeline ID, and a symbol "E" indicating "dedicated" attached to an end thereof. The dedicated timeline information ETLD includes the temporary patient ID, the in-hospital patient ID, the scheduled medical care item, transition information, dedicated registration information, and the status. For the transition information, when management of the medical care information transitions from the emergency timeline information FTLD to the dedicated timeline information ETLD, the medical care information registered in the emergency registration information of the emergency timeline information FTLD is registered. The medical care information of the patient P generated after the creation of the dedicated timeline information ETLD is registered in the dedicated registration information. The same items as those of the emergency timeline information FTLD are registered in the status.

As illustrated in Fig. 11, the emergency timeline screen FTLV created based on the emergency timeline information FTLD is distributed to each of client terminals 26 to 28, and displayed on the display of each of the client terminals 26 to 28. The emergency timeline screen FTLV distributed to the emergency portable terminal 26 or the medical specialist portable terminal 28 is operated by a touch operation in the touch panel. Further, the emergency timeline screen FTLV distributed to the in-hospital terminal 27 is operated by the keyboard or the mouse.

The emergency timeline screen FTLV includes the time bar 75 indicating a flow of time in a direction from the left to the right, a plurality of lead lines 76 extending downward from the time bar 75, and a plurality of information display frames 77 having rectangular frame shape at tips of the lead lines 76. A portion or a reduced image of the medical care information is displayed within each information display frame 77. A name of a transmission source of the medical care information such as a name of a medical device or a hospital is displayed under each information display frame 77. A transmission time of each piece of medical care information is displayed next to the lead line 76 under the time bar 75. When the information display frame 77 is selected in the emergency timeline screen FTLV, the medical care information corresponding to the selected information display frame 77 is read from the emergency timeline information FTLD and displayed on the emergency timeline screen FTLV.

A scheduled medical care item 78 is displayed above the time bar 75. Since the treatment available time has not been set in the scheduled medical care item of the emergency timeline information FTLD as described above, the scheduled medical care item 78 is not arranged along the time bar 75, but is displayed together above the time axis.

The timeline ID, the temporary patient ID, and gender and age of the patient P included in the dispatch information received from the fire emergency system are displayed to the upper left of the emergency timeline screen FTLV. An elapsed time from creation of the emergency timeline information FTLD, and a current time are displayed to the upper center and to the right of the emergency timeline screen FTLV.

A termination button 80 to be operated to terminate the operation of the emergency timeline information FTLD when the timeline information is not required due to a condition of the patient being mild or when the emergency timeline information FTLD is not required due to patient's recovery, death, or the like is provided to the lower left of the emergency timeline screen FTLV. When the termination button 80 is operated, an operation termination registration screen for inputting reasons for termination and registering the operation termination is displayed on the timeline screen.

A transport completion button 82 arranged to the right of the termination button 80 is operated by the paramedic F after the transport to the hospital of the patient P has been completed. The transport completion button 82 is displayed only in the emergency timeline screen FTLV of the emergency portable terminal 26 such that an inadvertent operation of a person other than the paramedic F is prevented. When the transport completion button 82 is operated, the emergency portable terminal 26 transmits transport completion information to the medical support server 21.

A transport situation display portion 84 in which a transport status of the patient P is displayed is provided to the right in a title portion 83 provided in an upper portion of the timeline screen FTLV. The timeline management unit 50 displays "transporting" in the transport situation display portion 84 at the time of creation of the emergency timeline information FTLD, and changes the transport situation display portion 84 into "transported" when the transport complete information is input from the emergency portable terminal 26.

A disease specifying button 86 arranged to the right of the transport completion button 82 is operated by the doctor D in the hospital when the disease of the patient P has been specified in the hospital after completion of the transport of the patient P to the hospital. The disease specifying button 86 is displayed only in the emergency timeline screen FTLV of the in-hospital terminal 27 in the hospital to which the patient P has been transported, so as to prevent an inadvertent operation of persons other than the doctor D of the hospital to which the patient P has been transported. For the in-hospital terminal 27, when the disease specifying button 86 is operated, a disease name input window for inputting a disease name is displayed on the emergency timeline screen FTLV.

A comment button 85 to be operated when the comment is transmitted to the emergency timeline information FTLD is provided to the lower right of the emergency timeline screen FTLV. When the comment button 85 is operated, a comment input window for inputting the comment is displayed on the emergency timeline screen FTLV.

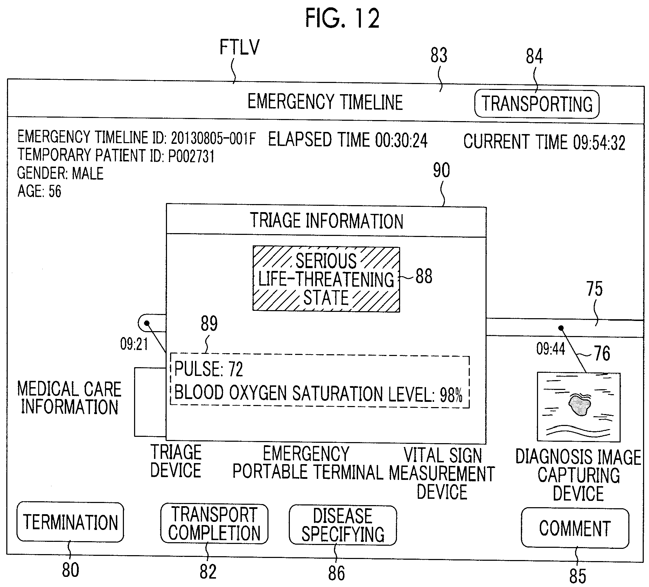

Fig. 12 illustrates a case in which the information display frame 77 of the triage device 22 is selected in the emergency timeline screen FTLV. A sub-window 90 in which a description sentence 88 of the degree of severity in which a background color is the identification color indicating the degree of severity, and a measurement result 89 of a pulse and a blood oxygen saturation level are displayed is displayed on the emergency timeline screen FTLV.

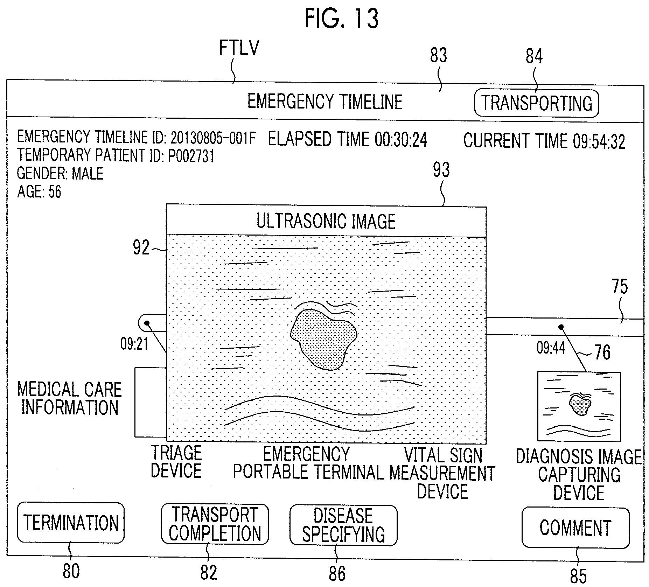

Fig. 13 illustrates a state in which the information display frame 77 of the diagnosis image capturing device 24 has been selected in the emergency timeline screen FTLV. A sub-window 93 on which an ultrasonic image 92 is displayed is displayed on the emergency timeline screen FTLV.

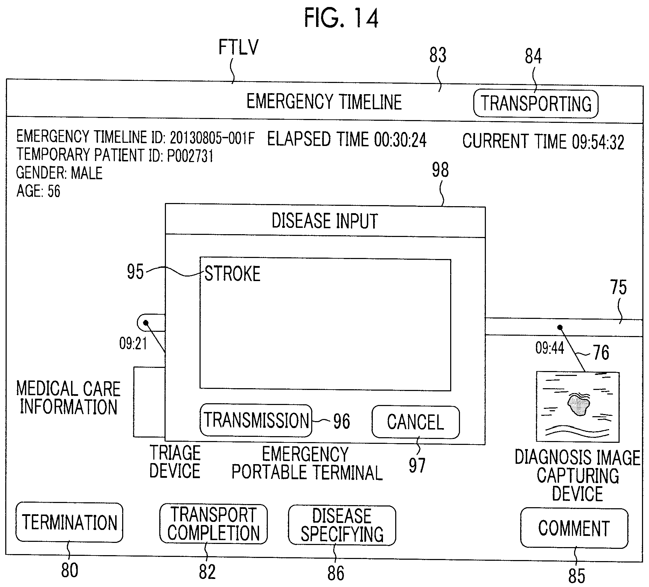

Fig. 14 illustrates the case in which the disease specifying button 86 is operated in the emergency timeline screen FTLV. A disease input window 98 in which a disease name input field 95, a transmission button 96, and a cancel button 97 are provided is displayed on the emergency timeline screen FTLV.

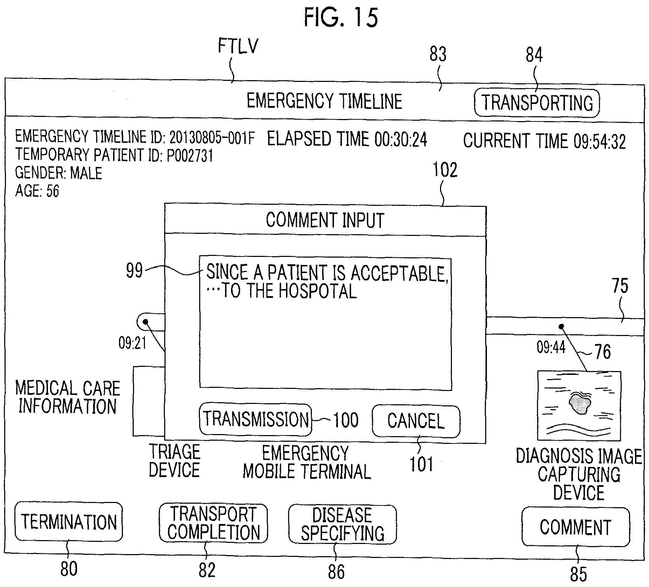

Fig. 15 illustrates a case in which the comment button 85 has been operated in the emergency timeline screen FTLV. A comment input window 102 in which a comment input field 99, a transmission button 100, and a cancel button 101 are provided is displayed on the emergency timeline screen FTLV.

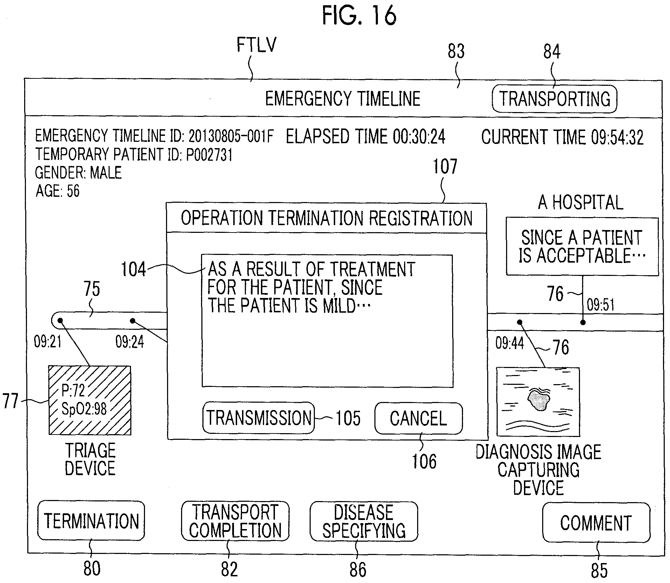

Fig. 16 illustrates a case in which the termination button 80 is operated in the emergency timeline screen FTLV. An operation termination registration window 107 in which a termination reason input field 104 in which a reason for termination of the operation of the timeline information is input, a transmission button 105, and a cancel button 106 are provided is displayed on the emergency timeline screen FTLV. In the operation termination registration window 107, when the reason for termination is not input to the termination reason input field 104, the timeline termination information is not transmitted to the medical support server 21 even when the transmission button 105 is operated. Accordingly, even when the termination button 80 is erroneously operated, the timeline information is not immediately terminated. The operation termination registration window 107 is also displayed even when the termination button 80 has been operated in the dedicated timeline screen ETLV.

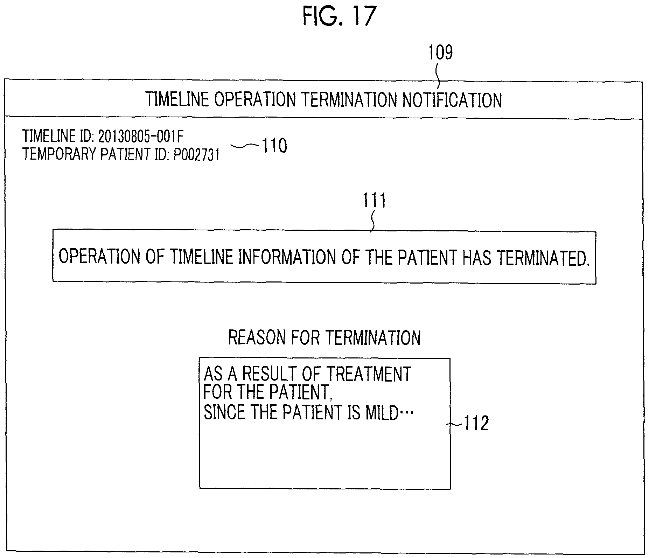

Fig. 17 illustrates an operation termination screen 109 to be distributed to the client terminals 26 to 28 after the operation termination of the timeline information. The timeline ID and a temporary patient ID 110, a message 111 indicating that the operation of the timeline information terminates, and the reason for termination 112 input in the operation termination registration window 107 are displayed in the operation termination screen 109.

As illustrated in Fig. 18, the dedicated timeline screen ETLV created based on the dedicated timeline information ETLD is distributed to the respective client terminals 26 to 28. The dedicated timeline screen ETLV is displayed on the display of each of the client terminals 26 to 28. The dedicated timeline screen ETLV is operated by the touch panel or the mouse, similar to the emergency timeline screen FTLV.

The time bar 75, the lead line 76, and the information display frame 77 are included in the dedicated timeline screen ETLV, similar to the emergency timeline screen FTLV. For example, "stroke" is displayed as a disease name 115 of the patient P in the title portion 83 of the dedicated timeline screen ETLV.

Further, the plurality of schedule display frames 117 having rectangular frame shapes are arranged at tips of the lead lines 76 over the time bar 75. The lead line 76 of each schedule display frame 117 is drawn from a treatment available time which is a sum of a time at which disease of the patient P is generated, an emergency notification time, or a creation time of the emergency timeline information FTLD and the treatment available time of the scheduled medical care item described in the schedule display frame 117. Next to the lead line 76, the treatment available time of each scheduled medical care item is displayed. Accordingly, by viewing the dedicated timeline screen ETLV, it is possible to confirm not only the medical care information of the patient P, but also the scheduled medical care item and the treatment- allowed time of the scheduled medical care item.

Since other portions of the dedicated timeline screen ETLV have the same configurations and functions as those of the emergency timeline screen FTLV, detailed description thereof will be omitted. In the dedicated timeline screen ETLV, it is not necessary to perform completion of the transport of the patient P to the hospital and specifying of the disease, the transport completion button 82 and the disease specifying button 86 are not displayed in the dedicated timeline screen ETLV.

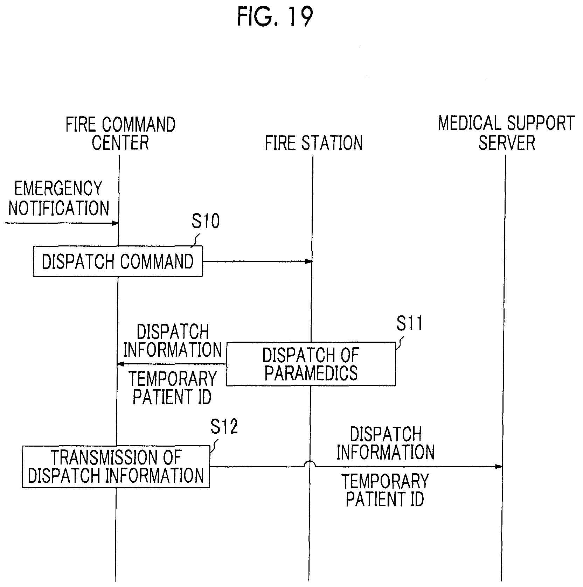

Next, an operation of the above embodiment will be described. As illustrated in Fig. 19, the fire command center 10 receives an emergency notification from the patient P or the like, and specifies an address of the site 15 from content of the notification or the like. The fire emergency system of the fire command center 10 imparts the temporary patient ID to the patient P, and transmits a dispatch command including this temporary patient ID to the fire station 11 (S10).

The fire station 11 organizes paramedics in response to the dispatch command, and dispatches the ambulance 16 (S11). The dispatch processing system of the fire station 11 transmits dispatch information including device information of each of the medical devices 22 to 25 mounted on the ambulance 16, terminal information of the emergency portable terminal 26 of each paramedic F to dispatch, and the temporary patient ID to the fire command center 10. The fire emergency system of the fire command center 10 transmits the dispatch information and the temporary patient ID to the medical support server 21 over the communication network 30 (S12).

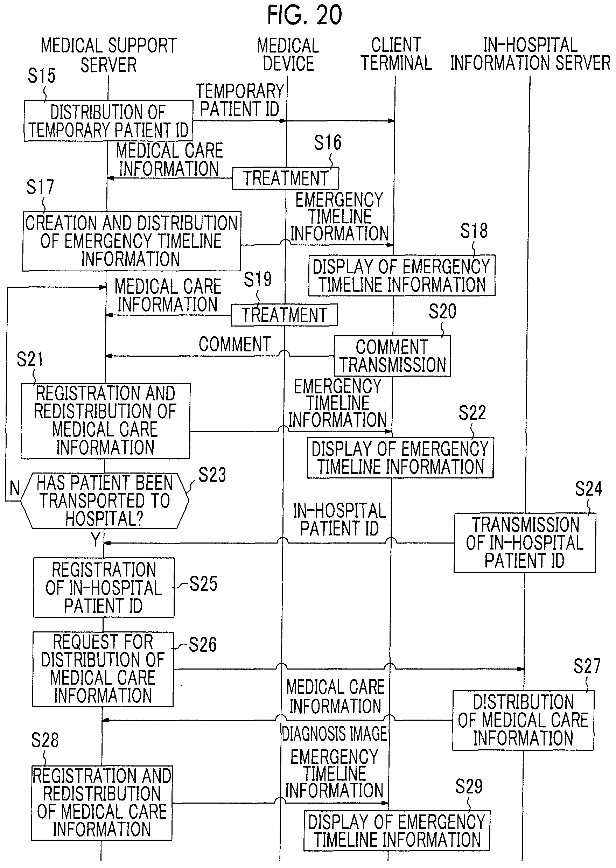

As illustrated in Fig. 20, the patient ID distribution unit 49 of the medical support server 21 distributes the temporary patient ID to each of the medical devices 22 to 25 and each emergency portable terminal 26 based on the communication information included in the dispatch information. Further, the patient ID distribution unit 49 also distributes the temporary patient ID to each in-hospital terminal 27 and each medical specialist portable terminal 28 based on the distribution destination DB 54 (S15).

The paramedics arriving at the site 15 starts medical care of the patient P. The paramedic F brings out the triage device 22 from the ambulance 16, mounts the triage device 22 on the body of the patient P, and begins to set the degree of severity and measure vital signs (S16). The triage device 22 transmits triage information Td including the degree of severity, the result of vital sign measurement, and the temporary patient ID as medical care information from the communication unit 60 to the medical support server 21 (S16).

The treatment start information reception unit 50a receives first medical care information acquired from the patient P and, in this case, the triage information. The triage information first received by the treatment start information reception unit 50a is handled as the treatment start information. The timeline creation unit 50b starts creation of the emergency timeline information FTLD corresponding to the degree of severity of the triage information in response to reception of the treatment start information (S17). The timeline creation unit 50b registers the received medical care information and the temporary patient ID in the format FFD read from the storage device 44 to create the timeline emergency information FTLD, imparts a timeline ID to the emergency timeline information FTLD, and stores the resultant emergency timeline information FTLD in the timeline DB 53.

When the medical device that first acquires the medical care information from the patient P is the vital sign measurement device 23, the emergency timeline information FTLD is created according to the vital information of the vital sign measurement device 23. Further, when the medical device that first acquires the medical care information from the patient P is the diagnosis image capturing device 24, the emergency timeline information FTLD is created according to the ultrasonic image information of the diagnosis image capturing device 24. Similarly, when the medical device that first acquires the medical care information from the patient P is the in-vehicle camera 25, the emergency timeline information FTLD is created according to the in-vehicle imaging data of the in-vehicle camera 25.

The distribution unit 51 reads the emergency timeline information FTLD stored in the timeline DB 53 and creates the emergency timeline screen FTLV based on the read emergency timeline information FTLD. The distribution unit 51 distributes the created emergency timeline screen FTLV to the client terminals 26 to 28 based on the distribution information stored in the distribution destination DB 54 (S17). The emergency portable terminal 26, the in-hospital terminal 27, and the medical specialist portable terminal 28 receive the distributed emergency timeline screen FTLV and display the emergency timeline screen FTLV on the display (S18).

The paramedic F, the doctor D, and the medical specialist S can view the emergency timeline screen FTLV displayed on the display. Further, when confirmation of details of the medical care information on the emergency timeline screen FTLV is desired, the information display frame 77 is selected. Then, as illustrated Figs. 12 and 13, a sub-window in which the selected medical care information is displayed is displayed on the emergency timeline screen FTLV.

The paramedic F continues to perform the treatment of the patient P, even after the first treatment that is a creation trigger of the emergency timeline information FTLD is performed (S19). The paramedic F photographs the site 15, the state of the patient P at the site 15, or the like using the emergency portable terminal 26. Further, the paramedic F records an interview of the patient P or a witness using the emergency portable terminal 26. The paramedic F transports the patient P into the ambulance 16 using a stretcher or the like, and then, continues to perform the treatment of the patient P using the vital sign measurement device 23 or the diagnosis image capturing device 24. Further, the in-vehicle camera 25 photographs a state of the patient P in the ambulance 16.

The triage device 22, the vital sign measurement device 23, the diagnosis image capturing device 24, the in-vehicle camera 25, and the emergency portable terminal 26 used for treatment of the patient P transmit the triage information, the vital information, the ultrasonic image data, the in-vehicle imaging data, and portable imaging data to which the temporary patient ID has been assigned, as the medical care information, to the medical support server 21 (S19).

When the paramedic F has a question or the like for the treatment of the patient P, the paramedic F calls for the comment input window 102 on the emergency timeline screen FTLV of the emergency portable terminal 26 as illustrated in Fig. 15, and inputs the question to the comment input field 99. Further, when the doctor D and the medical specialist S perform answering a question of the paramedic F, providing advice or an opinion, asserting acceptance of the patient P, or the like, the doctor D and the medical specialist S input such comments to the comment input window 102. The comment input to the comment input window 102 is transmitted to the medical support server 21 as the medical care information (S20). Accordingly, the paramedic F can obtain the answer to the question from the emergency timeline information FTLD, information on whether the patient is acceptable, or the like.

When the medical care information registration unit 50c receives the medical care information from each of the medical devices 22 to 25 and each of the client terminals 26 to 28 after the emergency timeline information FTLD is created, the medical care information registration unit 50c confirms the temporary patient ID in the received medical care information, and registers the received medical care information in the emergency timeline information FTLD corresponding to the temporary patient ID (S21) . The distribution unit 51 redistributes the emergency timeline information FTLD in which the medical care information has been registered, to each of the client terminals 26 to 28 (S21). The emergency portable terminal 26, the in-hospital terminal 27, and the medical specialist portable terminal 28 display the redistributed emergency timeline screen FTLV on the display (S22).

When the patient P is transported to any one of the hospitals 12A to 12C, the transport completion button 82 in the emergency timeline screen FTLV is operated by the paramedic F. When the transport completion button 82 is operated, the emergency portable terminal 26 transmits transport completion information to the medical support server 21. The medical care information registration unit 50c confirms the completion of the transport of the patient P to the hospital by receiving the transport completion information. The medical care information registration unit 50c changes the display of the transport situation display portion 84 of the emergency timeline screen FTLV and the dedicated timeline screen ETLV from "transporting" to "transported".

The in-hospital information server 29 imparts the in-hospital patient ID to the transported patient P, and transmits this in-hospital patient ID to the medical support server 21 (S24). The medical care information registration unit 50c registers the in-hospital patient ID in the emergency timeline information FTLD of the patient P (S25).

When the patient P is transported to the hospital (YES in S23), the medical care information registration unit 50c designates the in-hospital patient ID of the patient P and requests the in-hospital information server 29 of the hospital to which the patient P has been transported, to distribute the medical care information and the diagnosis image (S26). In response to the distribution request, the in-hospital information server 29 reads the medical care information of the patient P from the electronic medical chart DB, reads the diagnosis image of the patient P from the diagnosis image DB, and distributes the medical care information and the diagnosis image to the medical support server 21 as medical care information (S27). The medical care information registration unit 50c registers the medical care information and the diagnosis image distributed from the in-hospital information server 29 in the emergency timeline information FTLD (S28). The distribution unit 51 redistributes the emergency timeline screen FTLV to the in-hospital terminal 27 and the medical specialist portable terminal 28 based on the emergency timeline information FTLD in which the medical care information has been registered (S28). The in-hospital terminal 27 and the medical specialist portable terminal 28 display the redistributed emergency timeline screen FTLV on the display (S29).

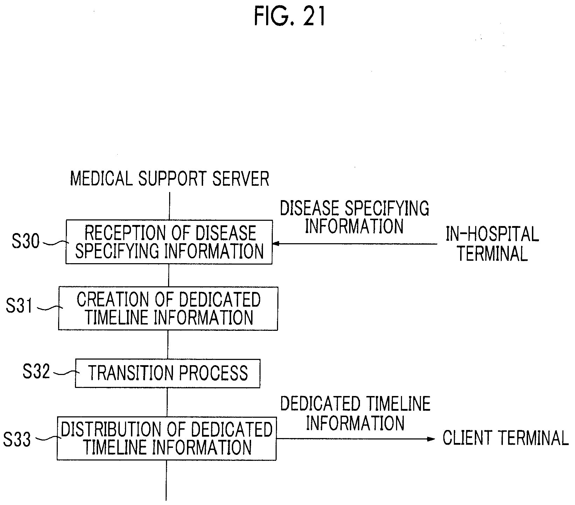

When the disease of the patient P is specified, the doctor D in the hospital to which the patient P has been transported operates the disease specifying button 86 in the emergency timeline screen FTLV displayed on the in-hospital terminal 27. When the disease specifying button 86 is operated, the in-hospital terminal 27 transmits disease specifying information including the disease name to the medical support server 21. As illustrated in Fig. 21, the disease specifying information reception unit 50e receives the disease specifying information transmitted from the in-hospital terminal 27 (S30), and the timeline creation unit 50b creates one piece of dedicated timeline information ETLD for each temporary patient ID imparted to the disease specifying information (S31).

The timeline creation unit 50b reads a dedicated format EFD that is a template of the dedicated timeline information ETLD corresponding to the disease name included in the disease specifying information from the format DB 52, and registers the temporary patient ID in the read dedicated format EFD to create the dedicated timeline information ETLD. For example, the timeline creation unit 50b creates the dedicated timeline information ETLD corresponding to cerebrovascular disease when the disease name is stroke, and creates the dedicated timeline information ETLD corresponding to cardiovascular disease when the disease name is myocardial infarction. The timeline management unit 50 imparts a dedicated timeline ID to the created dedicated timeline information ETLD, and stores the resultant dedicated timeline information ETLD in the timeline DB 53.

The medical care information transition unit 50f makes a transition of the management of the medical care information registered in the emergency timeline information FTLD to the dedicated timeline information ETLD having the same temporary patient ID (S32). The dedicated timeline information ETLD manages the medical care information transitioning from the emergency timeline information FTLD along a time axis. The distribution unit 51 reads the dedicated timeline information ETLD stored in the timeline DB 53, and creates the dedicated timeline screen ETLV based on the read dedicated timeline information ETLD. The distribution unit 51 distributes the dedicated timeline screen ETLV to the in-hospital terminal 27 and the medical specialist portable terminal 28 based on the distribution information stored in the distribution destination DB 54 (S33). The distributed dedicated timeline screen ETLV is displayed on the display of the in-hospital terminal 27 and the medical specialist portable terminal 28.

The timeline management unit 50 operates the dedicated timeline information ETLD, similar to the emergency timeline information FTLD. Specifically, the medical care information registration unit 50c registers the medical care information and the diagnosis image of the patient P distributed from the in-hospital information server 29 in the hospital to which the patient P has been transported, and the comment transmitted from the in-hospital terminal 27 and the medical specialist portable terminal 28 in the dedicated timeline information ETLD as the medical care information, and redistributes the dedicated timeline information ETLD in which the medical care information has been registered, to the in-hospital terminal 27 and the medical specialist portable terminal 28.

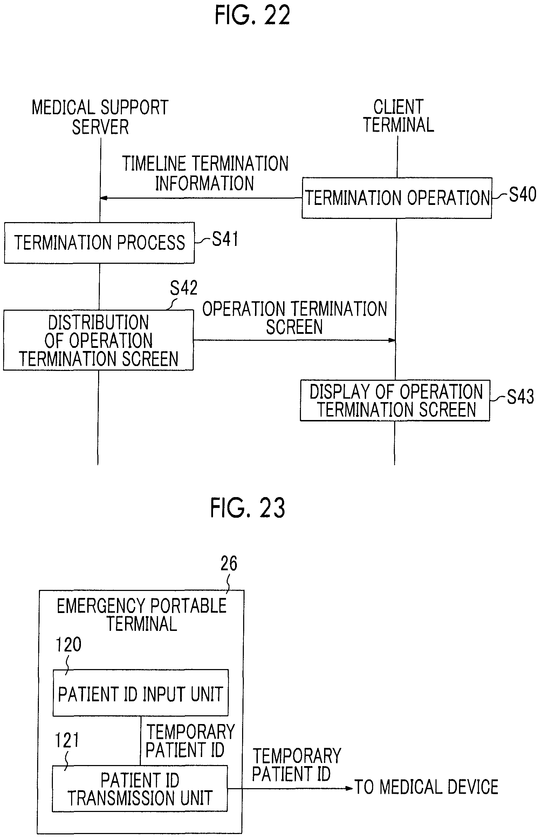

When the timeline information is not required due to a condition of the patient being mild or when the timeline information is not required due to patient's recovery, death, or the like, the termination button 80 of the emergency timeline screen FTLV or the dedicated timeline screen ETLV is operated in the client terminals such as the medical support server 21, the emergency portable terminal 26, the in-hospital terminal 27 and the medical specialist portable terminal 28. The operation termination registration window 107 illustrated in Fig. 16 is displayed on the display of the terminal in which the termination button 80 has been operated. As illustrated in Fig. 22, when the reason for termination is input in the operation termination registration window 107 and the transmission button 105 is operated, the timeline termination information is transmitted to the medical support server 21 (S40).

The timeline termination unit 50d receiving the timeline termination information starts the termination process (S41). In this termination process, "operation termination" is registered as status information in the status of the emergency timeline information FTLD or the dedicated timeline information ETLD of the patient that is a terminating target, and a reason for operation termination is also recorded. For the emergency timeline information FTLD or the dedicated timeline information ETLD in which the status is "operation termination", the registration of the medical care information then terminates. When the operation of the timeline information terminates in the timeline termination unit 50d, the distribution unit 51 distributes the operation termination screen 109 illustrated in Fig. 17 to the client terminals 26 to 27 (S42). The operation termination screen 109 is displayed on the displays of the client terminals 26 to 28 (S43). Accordingly, the paramedic F, the doctor D, and the medical specialist S can recognize that the operation of the emergency timeline information FTLD or the dedicated timeline information ETLD of the patient has terminated.

As described above, according to the medical support system 19 of this embodiment, since the general-purpose emergency timeline information FTLD is created in the initial step of emergency, the medical care information in the initial step of emergency can be registered in the emergency timeline information FTLD. Further, since it is possible to confirm the medical care information in the initial step of emergency and the scheduled medical care item required in the initial step of emergency by viewing the emergency timeline information FTLD, it is possible to appropriately perform medical care on the patient in the initial step of emergency.

Further, after the disease of the patient has been specified, the dedicated timeline information ETLD of the specified disease only is created and the medical care information registered in the emergency timeline information FTLD transitions to the dedicated timeline information ETLD. Accordingly, by viewing the dedicated timeline information ETLD, it is possible to confirm the medical care information of the patient after specifying of the disease from initial step of emergency and the scheduled medical care item corresponding to the disease. Thus, it is possible to appropriately perform medical care on the patient according to the disease.

Since the medical care item performed in common in the initial step of emergency of a plurality of types of diseases is set as the scheduled medical care item in the emergency timeline information FTLD, it is possible to confirm the medical care item necessary in the initial step of emergency for any disease. Further, since the medical care item required to be performed for each type of disease is set as the scheduled medical care item in the dedicated timeline information ETLD, it is possible to confirm the medical care item required for medical care for each disease.

Further, while in this embodiment, the timeline information of the patient is necessarily created when the paramedic F is dispatched, the operation of the timeline information can terminate when the timeline information is not required due to a condition of the patient being mild or when the timeline information is no longer required due to patient' s recovery, death, or the like. Accordingly, it is possible to prevent confusion or incorrect medical care from being caused by a continuous operation of the unnecessary timeline information, and to reduce a load on the medical support server 21.

While in the above embodiment, the emergency timeline information FTLD has been created according to the medical care information first acquired from the patient P in the medical devices such as the triage device 22, the vital sign measurement device 23, the diagnosis image capturing device 24, and the in-vehicle camera 25. On the other hand, when a type of medical device first used in the initial step of emergency is determined in advance, the emergency timeline information FTLD may be created according to medical care information that such a type of medical device has first acquired from the patient P. For example, among the medical devices such as the triage device 22, the vital sign measurement device 23, the diagnosis image capturing device 24, and the in-vehicle camera 25, the likelihood of the triage device 22 being initially brought out and used in the site is high, and accordingly, the emergency timeline information FTLD may be created based on the triage information of the triage device 22.

In the above embodiment, since the temporary patient ID is distributed from the medical support server 21 to each of the medical devices 22 to 25 and each of the client terminals 26 to 28 when the paramedics are dispatched, only one type of temporary patient ID can be set. However, even when the number of patients is one at the time of the dispatch, it may be found that there is a plurality of patients after the paramedics arrive at the site. In this case, since one type of temporary patient ID is imparted to the plurality of patients and the same emergency timeline information FTLD is used, this may cause medical error.

In order to be able to cope with such a case, the patient ID input unit 120 that manually inputs the temporary patient ID using a touch panel or the like, and the patient ID transmission unit 121 that individually transmits the input temporary patient ID to each medical device may be provided in the emergency portable terminal 26, as illustrated in Fig. 23. Accordingly, even when it is found that there is a plurality of patients at the site, it is possible to impart the temporary patient ID to each patient, and thus, to create the timeline information for each patient.

### [Second Embodiment]

Next, a second embodiment of the present invention will be described. This embodiment relates to a medical support system that creates a plurality of dedicated timeline information ETLD to correspond to one piece of the emergency timeline information. The same configurations as those in the first embodiment are denoted with the same reference numerals, and detailed description thereof will be omitted.

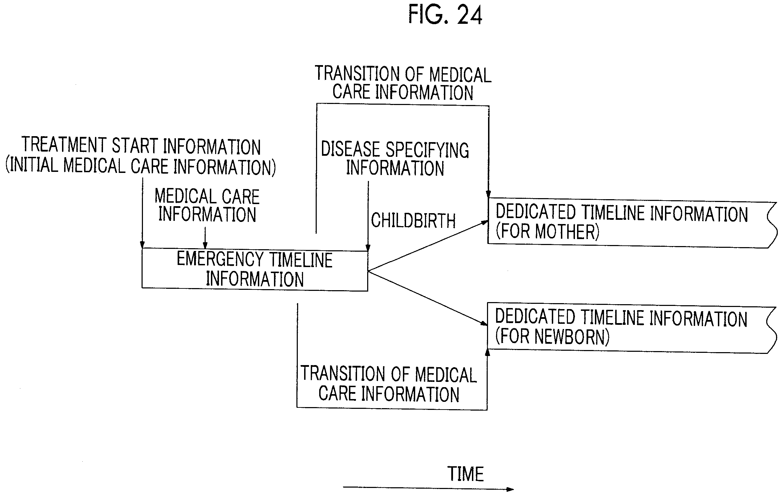

As illustrated in Fig. 24, the emergency timeline information FTLD is created by the timeline creation unit 50b of the medical support server 21 according to the treatment start information. The medical care information of the patient generated in the initial step of emergency is registered in the emergency timeline information FTLD in each case. When the disease of the patient is specified as childbirth based on the disease specifying information, the timeline creation unit 50b creates two pieces of dedicated timeline information ETLD for a mother and a newborn, and makes a transition of the medical care information registered in the emergency timeline information FTLD to each pieces of dedicated timeline information ETLD. Accordingly, since the medical care information for the mother and the newborn managed together before the childbirth can be managed using the respective dedicated timeline information after the childbirth, optimal medical care can be performed on the mother and the newborn.

### [Third Embodiment]

Next, a third embodiment of the present invention will be described. This embodiment relates to a medical support system that transfers all or part of a function of managing timeline information in a medical support server to another server after an initial step of emergency ends, that is, after a patient P is transported to a hospital. The same configurations as those in the first embodiment are denoted with the same reference numerals, and detailed description is omitted.

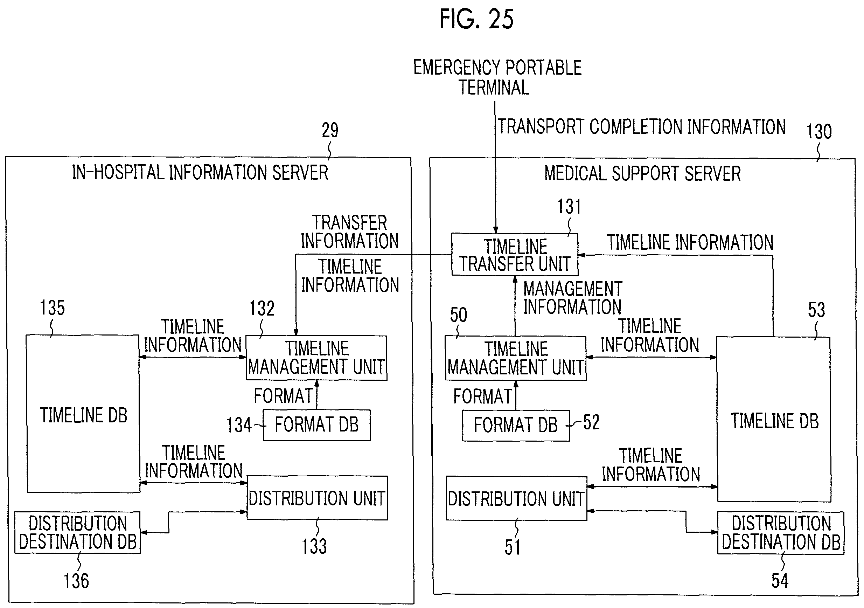

As illustrated in Fig. 25, the medical support server 130 of this embodiment includes a timeline transfer unit 131 that transfers all or part of the function of managing the timeline information to another server. A server to which the function of managing the timeline information is transferred is, for example, the in-hospital information server 29 of the hospital to which the patient P is transported. The in-hospital information server 29 includes a timeline management unit 132 having the same function as that of the medical support server 130, a distribution unit 133, a timeline DB 134, a timeline DB 135, and a distribution destination DB 136.

When the transport completion button 82 is operated in the emergency timeline screen FTLV of the emergency portable terminal 26, the transport completion information is input from the emergency portable terminal 26 to the timeline transfer unit 131. The transport completion information is information indicating completion of the transport of the patient P to the hospital, as described in the first embodiment.

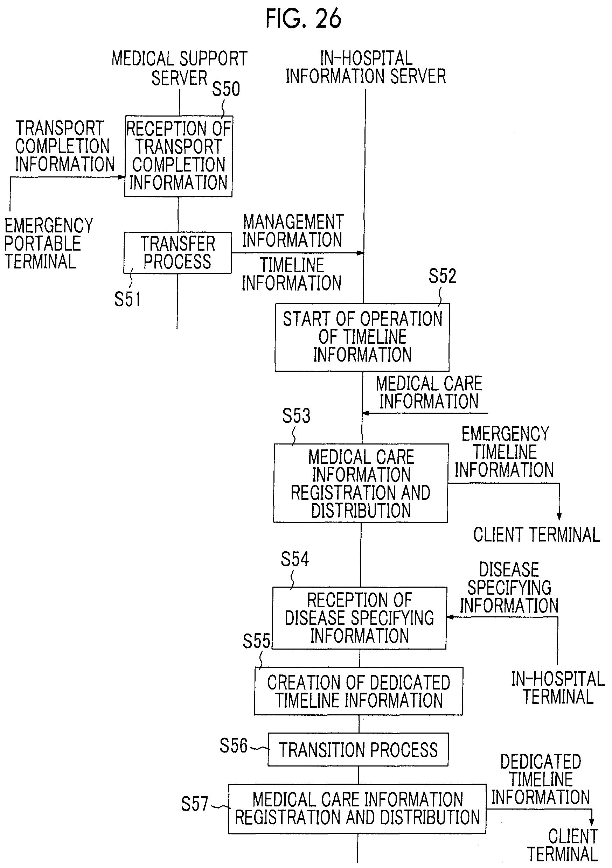

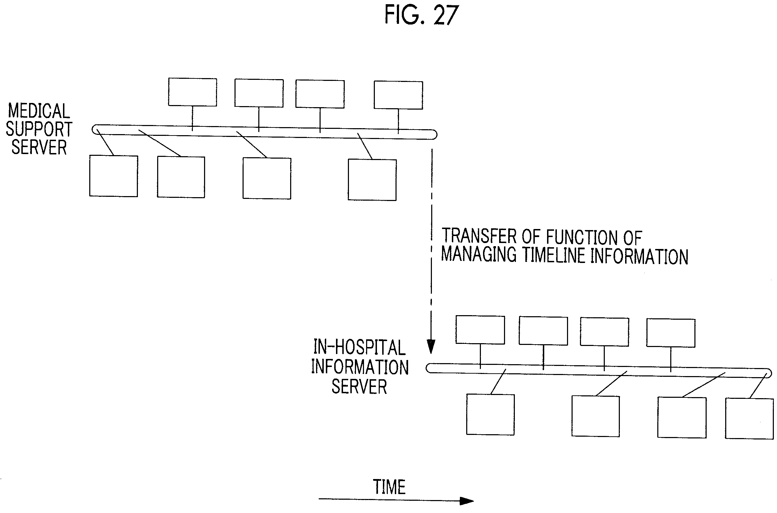

As illustrated in Fig. 26, when the transport completion information is input(S50), the timeline transfer unit 131 executes a transfer process of transferring the management of the emergency timeline information FTLD from the medical support server 130 to the in-hospital information server 29 (S51). In this transfer process, the timeline transfer unit 131 transmits the emergency timeline information FTLD of the patient P and transfer information indicating that the emergency timeline information FTLD of the patient P is transferred, to the timeline management unit 132 of the in-hospital information server 29. The timeline management unit 132 of the in-hospital information server 29 confirms that the emergency timeline information FTLD is transferred based on the transfer information, and stores the received emergency timeline information FTLD in the timeline DB 135. The timeline management unit 132 starts the operation of the emergency timeline information FTLD of the patient P (S52), and performs the registration of the medical care information in the emergency timeline information FTLD and the distribution of the emergency timeline information FTLD to the client terminal (S53), as illustrated in Fig. 27.

Further, when the timeline management unit 132 of the in-hospital information server 29 receives the disease specifying information of the patient P transmitted from the in-hospital terminal 27 (S54), the timeline management unit 132 creates the dedicated timeline information ETLD corresponding to the disease name included in the disease specifying information (S55), and makes a transition of the medical care information from the emergency timeline information FTLD to the dedicated timeline information ETLD (S56). The timeline management unit 132 starts the operation of the dedicated timeline information ETLD of the same patient P, and performs registration of the medical care information in the dedicated timeline information ETLD and distribution of the timeline information to the client terminal, similar to the emergency timeline information FTLD (S57).

According to this embodiment, since a function of managing the timeline information of the medical support server is transferred to another server after the patient is transported to the hospital, it is possible to reduce a load on the medical support server that is used in a wide medical service zone of each administrative district.

While, in the third embodiment, functions of the storage of the timeline information, the registration of the medical care information, and the distribution of the timeline information have been transferred to the in-hospital information server 29, at least one of these may be transferred.

While, in each embodiment, the medical support server has been installed in the fire command center, the medical support server may be installed in an emergency medical center or a data center within the medical service zone, a data center outside the medical service zone, or the like. Further, while authorization to manage the timeline information has been transferred after the transport of the patient to the hospital is completed, the authorization may be transferred after a transport destination hospital is determined.

While a triage device that sets the degree of severity through a touch panel has been used as the triage device, for example, a triage tag in which a circuit in which a resistance or the like is changed according to a position at which the tag is broken, such that the degree of severity can be set, is provided in the tag having the same appearance as that of a conventional triage tag made of paper may be used.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

## Claims

1. A medical support server (21) that supports medical care for a patient (P) based on timeline information for managing medical care information of the patient and a scheduled medical care item required for medical care of the patient along a time axis, the medical support server comprising:
a timeline creation unit (50b) for creating, as the timeline information, emergency timeline information (FTLD) used in an initial step of emergency in which the patient is transported from a site (15) as a transport source to a medical facility (12A, 12B, 12C) as a transport destination, and creating dedicated timeline information (ETLD) of specified disease of the patient in the case where the disease of the patient is specified; and
a medical care information transition unit (50f) for making a transition of management of the medical care information of the patient from the emergency timeline information to the dedicated timeline information in the case where the dedicated timeline information is created after the creation of the emergency timeline information of the patient.

2. The medical support server according to claim 1, wherein in the emergency timeline information, a medical care item performed in common in the initial step of emergency of a plurality of types of diseases is set as the scheduled medical care item.

3. The medical support server according to claim 2, wherein a medical care item that is treated by a paramedic (F) is set in the scheduled medical care item of the emergency timeline information.

4. The medical support server according to claim 1, wherein in the dedicated timeline information, an item of medical care to be performed for each type of disease is set as the scheduled medical care item.

5. The medical support server according to claim 4, wherein, in the scheduled medical care item of the dedicated timeline information, at least one of a type and content of a medical care item is different according to the type of the disease.

6. The medical support server according to claim 5, wherein a plurality of types of dedicated timeline information in which thrombolytic therapy is set in common, as the scheduled medical care item, includes a treatment available time of the thrombolytic therapy and a dose of a thrombolytic drug different according to a type of disease.

7. The medical support server according to claim 6, wherein the plurality of types of dedicated timeline information include at least first dedicated timeline information corresponding to cerebrovascular disease, and second dedicated timeline information corresponding to cardiovascular disease.

8. The medical support server according to claim 1, wherein in the case where the emergency timeline information is used for childbirth, the timeline creation unit creates, as the dedicated timeline information, maternal timeline information used for management of the medical care information of a mother, and neonatal timeline information used for management of the medical care information of a newborn.

9. The medical support server according to claim 1, wherein the timeline creation unit creates the emergency timeline information and the dedicated timeline information based on an emergency format in which a medical care item to be performed in common in the initial step of emergency of a plurality of types of diseases is set as the scheduled medical care item, and a plurality of types of dedicated formats in which a medical care item to be performed for each type of disease is set as the scheduled medical care item.

10. The medical support server according to claim 1, further comprising a medical care information registration unit (50c) for receiving the medical care information of the patient and registering the received medical care information in the emergency timeline information or the dedicated timeline information.

11. The medical support server according to claim 10, wherein the medical care information registration unit receives the medical care information transmitted from a medical device (22, 23, 24, 25) used by a paramedic (F) to treat the patient in the initial step of emergency, and registers the received medical care information in the emergency timeline information.

12. The medical support server according to claim 10, wherein the medical care information registration unit receives the medical care information from an in-hospital information server (29) for managing medical care information of the patient in the medical facility to which the patient is transported after the initial step of emergency ends, and registers the received medical care information in the dedicated timeline information.

13. The medical support server according to claim 1, further comprising a distribution unit (51) for distributing the emergency timeline information and the dedicated timeline information to a client terminal (26,27,28) over a communication network (30).

14. The medical support server according to claim 1, further comprising a timeline transfer unit (131) for transferring all or part of a management function of managing the timeline information to another server after the initial step of emergency ends.

15. The medical support server according to claim 14, wherein the timeline transfer unit transfers a function of creating the dedicated timeline information among functions of the timeline creation unit and a function of the medical care information transition unit to the other server.

16. A medical support system (19) including a medical support server (21) that supports medical care for a patient (P) based on timeline information for managing medical care information of the patient and a scheduled medical care item required for medical care of the patient along a time axis, the medical support system comprising:
A. a medical support server (21) including:
a timeline creation unit (50b) for creating, as the timeline information, emergency timeline information (FTLD) used in an initial step of emergency in which the patient is transported from a site (15) as a transport source to a medical facility (12A, 12B, 12C) as a transport destination, and creating dedicated timeline information (ETLD) of specified disease of the patient in the case where disease of the patient is specified;
a medical care information transition unit (50f) for making a transition of management of the medical care information of the patient from the emergency timeline information to the dedicated timeline information in the case where the dedicated timeline information is created after the creation of the emergency timeline information of the patient; and
a timeline transfer unit (131) for transferring all or part of a management function of managing the timeline information to another server after the initial step of emergency ends; and
B. a transport destination server (29) to which all or part of the management function of the timeline information is transferred by the timeline transfer unit.

17. The medical support system according to claim 16, wherein the transport destination server (29) is an in-hospital information server (29) that is managed in a medical facility as the transport destination of the patient.
